(19)

**Europäisches Patentamt**
**European Patent Office**
**Office européen des brevets**

(11) **EP 4 477 136 A1**

(12) **EUROPEAN PATENT APPLICATION**

(43) Date of publication:
18.12.2024 Bulletin 2024/51

(21) Application number: 23179054.4

(22) Date of filing: 13.06.2023

(51) International Patent Classification (IPC):
*A61B 5/00* (2006.01)        *G06T 7/00* (2017.01)

(52) Cooperative Patent Classification (CPC):
**A61B 5/0088; A61B 5/0071; A61B 5/4547;**
**A61B 5/7267; G06T 7/0012;** A61B 2576/02

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **3Shape A/S**
**1060 Copenhagen K (DK)**

(72) Inventors:
• **VANNAHME, Christoph**
  **1060 Copenhagen K (DK)**
• **DA LIO, Beatrice**
  **1060 Copenhagen K (DK)**
• **WESTERGAARD, Philip Grabow**
  **1060 Copenhagen K (DK)**
• **ALALOUF, Daniella**
  **1060 Copenhagen K (DK)**

(74) Representative: **Guardian**
**IP Consulting I/S**
**Diplomvej, Building 381**
**2800 Kgs. Lyngby (DK)**

(54) **METHOD AND SYSTEM FOR DETECTION OF DENTAL PLAQUE**

(57)     A computer-implemented method for detecting dental plaque on a digital 3D model (101) of a dental situation is disclosed. The method comprises receiving, by a processor, at least a part of the digital 3D model (101) of the dental situation, wherein the at least part of the digital 3D model (101) comprises fluorescence information associated with fluorescence emitted from dental plaque present on teeth of the dental situation. The fluorescence information comprises a red (R) fluorescence signal value and a green (G) fluorescence signal value of at least one unit element of the at least part of the digital 3D model (101). Further, the method comprises detecting a dental plaque parameter (301) on the at least part of the digital 3D model (101) based on the fluorescence information, wherein the dental plaque parameter (301) represents pathogenic plaque present on teeth of the dental situation. Detecting the dental plaque parameter (301) comprises computing a function of the red (R) fluorescence signal value and the green (G) fluorescence signal value for the at least one unit element of the at least part of the digital 3D model (101). Additionally, the method comprises displaying the at least part of the digital 3D model and the detected dental plaque parameter (301).

FIG. 2

EP 4 477 136 A1

## Description

### Technical field

The disclosure relates to a computer-implemented method and system for detecting dental plaque in a digital 3D dental model of a patient's dentition.

### Background

Dental plaque, referred to also as plaque in this disclosure, is a dental condition manifesting itself in the form of a biofilm that forms on human teeth. Plaque biofilm is a sticky, acidic biofilm, made of broken-down food by bacteria present in the mouth. Plaque may be removed through regular dental cleanings, daily brushing and/or flossing. However, if left untreated, plaque can cause formation of caries, tartar, gingivitis, or other oral diseases. Therefore, timely detection and monitoring of plaque by dental practitioners are essential for preserving tooth structure and overall oral health of a patient.

Currently, clinical detection and diagnosis of plaque is based on direct visual and tactile inspections of teeth by dental practitioners. A disclosing agent, for example in form of a tablet, can be used to stain the plaque in a bright color, typically red or blue color. Certain disclosing agents contain several dyes which may stain plaque of different concentrations and age in different colors. For example, new plaque may be stained in red color and old, pathological plaque in blue color. It is common, in dental offices, to utilize the disclosing agent only with patients assessed as being at high-risk. This results in all patients not receiving a full oral health assessment.

Development of intraoral scanning techniques has been instrumental in the transition to modern digital dentistry. Use of an intraoral 3D scanner allows the dental practitioners to accurately and quickly capture intraoral situation of the patient which may then be visualized on a display as a digital 3D dental model. Obtained digital 3D dental model of patient's teeth may thus serve as a digital impression, offering numerous advantages over a classical physical impression. Overall efficiency of dental procedures is thereby improved as, for example, the created digital impressions can be used in a range of applications such as design of dental restorations or design of orthodontic treatments. The digital 3D dental model may in addition, be used for diagnostic purposes to detect and visualize to the user, for example the dental practitioner, presence of a dental condition such as plaque, but also caries, gingivitis, gingival recession and/or tooth wear.

There is a clear need to develop methods and systems which will enable plaque detection in the dental situation from a single digital 3D dental model of the dental situation. Dental practitioners are also in need of a solution to visualize the plaque on the digital 3D dental model to the patient in a manner which may resemble use of a common disclosing agent. This, in turn, would allow for developing timely dental hygiene improvements to preserve oral health of the patient.

The present disclosure addresses prospects to utilize capabilities of the intraoral 3D scanner, such as the capability of exciting and recording fluorescence emitted from dental plaque on the teeth and to detect dental plaque on the digital 3D model of the dental situation.

Beyond just presence of plaque in the dental situation, there is also a need for digitally determining plaque thickness, as well as type of plaque present, for example whether the plaque present is new and thereby less harmful or old, pathogenic plaque. Identification and visualization of regions with high-risk pathogenic plaque is especially desired.

### Summary

In an embodiment, a computer-implemented method for detecting dental plaque on a digital 3D model of a dental situation is disclosed, wherein the method comprises:

- receiving, by a processor, at least a part of the digital 3D model of the dental situation, wherein the at least part of the digital 3D model comprises fluorescence information associated with fluorescence emitted from dental plaque present on teeth of the dental situation,
- detecting a dental plaque parameter on the at least part of the digital 3D model based on the fluorescence information, wherein the dental plaque parameter represents pathogenic plaque present on teeth of the dental situation,
- displaying the at least part of the digital 3D model and the detected dental plaque parameter.

Expression "3D" throughout the present disclosure refers to a term "three-dimensional". Similarly, term "2D" refers to a term "two-dimensional". Term "digital 3D model of a dental situation" refers to a digital, three-dimensional, computer-generated representation of the patient's dental situation.

Such digital 3D model may accurately correspond to the actual dental situation so that dental objects like teeth, teeth surfaces, restorations and gingiva on the digital 3D model correspond to those of the dental situation.

The digital 3D model may be constructed by the processor, based on scan data collected in an intraoral scanning process in which an intraoral 3D scanner may be used to scan the patient's dental situation comprising teeth and gingiva.

The intraoral 3D scanner throughout the disclosure is also referred to as the intraoral scanner. The digital 3D model can be stored in a memory of a computer system, for example in a Standard Triangle Language (STL) format.

The digital 3D model can be received or accessed by the processor. The digital 3D model may usually be displayed on a display screen in form of a 3D mesh, representing surfaces of teeth and gingival tissue of the dental situation. The 3D mesh may be comprised of individual facets, for example triangular facets while each facet comprises, for example three mutually connected vertices. Alternatively, the digital 3D model may be displayed as a point cloud comprising points, a graph comprising nodes and edges, a volumetric representation comprising voxels, or any other suitable 3D representation form.

The method according to an embodiment may comprise receiving, by the processor, the digital 3D model of the dental situation, wherein the digital 3D model comprises fluorescence information associated with fluorescence emitted from dental plaque present on teeth of the dental situation.

The fluorescence information, in the context of the disclosure, may comprise a red (R) fluorescence signal value and a green (G) fluorescence signal value for at least one unit element of the at least part of the digital 3D model. The fluorescence information may be represented numerically in Red, Green, Blue (RGB) color space [R: 0-255; G: 0-255; B: 0]. No blue data component is available due to filtration of this component during scanning or during post-processing.

The fluorescence information may be recorded by means of the intraoral scanner which may be equipped with a light source capable of exciting fluorescent material in the dental situation, an image sensor capable of recording the emitted fluorescence, and a filter to block the excitation light from the sensor while transmitting the fluorescent light thereby allowing observance of fluorescence. Pathogenic dental plaque, present on teeth of the dental situation, is such fluorescent material emitting fluorescence when illuminated with the light source.

Therefore, the intraoral scanner, besides having an optical system to capture geometric information related to 3D space placing of objects in the dental situation, and color information of the dental situation, may also be equipped with means to capture fluorescence emitted from the teeth in the dental situation. This may be achieved by integrating, in the intraoral scanner, the light source that can emit light intended to excite fluorescence in parts of the dental situation, and the image sensor that can measure that emitted fluorescence. By scanning the dental situation with such an intraoral scanner, fluorescence information may be obtained.

This may be of particular interest for pathogenic plaque detection. Pathogenic plaque is acidic and may lead to formation of caries or may cause periodontal diseases if untreated. A characteristic of pathogenic plaque is that the bacteria present therein emit red fluorescence when illuminated by the light source with an emission spectrum of, for example, below 500 nanometers. The red fluorescence emitted by pathogenic plaque, for example expressed in color intensity values of Red, Green, Blue (RGB) color space, may be higher than a specific threshold which may be determined. The fluorescence information obtained may be in form of red (R) fluorescence signal value and/or green (G) fluorescence signal value per unit element, for example per vertex of the at least part of the digital 3D model. It is therefore one aim of the present invention to detect pathogenic plaque based on the fluorescence information.

Throughout the disclosure terms "dental plaque" and "plaque" are used interchangeably.

The fluorescence information may be represented in Red, Green, Blue (RGB) color space, for example with the red (R) fluorescence signal value and the green (G) fluorescence signal value. A blue (B) fluorescence signal value may be filtered out during the scanning process or afterwards, during signal processing stage. A blue light, for example with wavelength of 405 nanometers, may be used by the light source of the intraoral scanner to excite the fluorescent material.

A unit element of the at least part of the digital 3D model, in the context of the disclosure, relates to a single building block of the at least part of the digital 3D model, arranged in 3D space. The unit element may therefore be a vertex and/or a facet, if the at least part of the digital 3D model is represented as a triangular 3D mesh of facets. The unit element may be a point, if the at least part of the digital 3D model is represented as a point cloud. The unit element may be a node, if the at least part of the digital 3D model is represented as a graph. The unit element may be a voxel, if the at least part of the digital 3D model is represented as a volumetric representation. The unit element may be a pixel, if the fluorescence information is comprised in a 2D image wrapped around the at least part of the digital 3D model. The 2D image comprising fluorescence information in this case may be referred to as fluorescence texture.

The fluorescence information may, in an example, be comprised in each vertex and/or facet of the at least part of the digital 3D model represented as the 3D mesh.

The dental plaque parameter may be, in an example, plaque presence value. In this case, the dental plaque parameter may be a simple indicator whether or not the dental plaque, of any type, is present in at least one unit element of the at least part of the digital 3D model. The plaque presence value may be a flag "YES" or "NO" to indicate plaque presence in the at least one unit element of the at least part of the digital 3D model. Such information on plaque presence may be of use to determine plaque index, for example. Plaque index determination will be elaborated further in the disclosure.

The dental plaque parameter may be, alternatively or additionally, a pathogenic plaque presence value. In this case, besides a simple indication on plaque presence, information on type of plaque present in the at least one unit element of the at least part of the digital 3D model is given. In particular, pathogenic plaque presence may be determined. Thus, the dental plaque parameter may represent pathogenic plaque present on teeth of the dental situation.

It may be of particular relevance for dental practitioners to detect pathogenic plaque which may lead to other oral diseases such as caries or periodontal diseases, if untreated.

Consequently, information on the type of plaque present in the dental situation may be obtained from a single digital 3D model of the dental situation by interpreting the fluorescence information. Of particular clinical relevance is the information about high-risk pathogenic plaque accumulation in order to timely prevent development of other oral diseases. Pathogenic plaque may be detected by interpreting color intensity values of a red and/or green channel of the fluorescence information, for example in the Red, Green, Blue (RGB) color space, as will be described further. For example, pathogenic plaque may emit red fluorescence above specific thresholds when illuminated with a blue light source.

A computer-implemented method for detecting dental plaque on the digital 3D model of dental situation, according to an embodiment may comprise:

- receiving, by the processor, the at least part of the digital 3D model of the dental situation, wherein the at least part of the digital 3D model comprises fluorescence information associated with fluorescence emitted from dental plaque present on teeth of the dental situation, wherein the fluorescence information comprises the red (R) fluorescence signal value and the green (G) fluorescence signal value of the at least one unit element of the at least part of the digital 3D model,
- detecting the dental plaque parameter on the at least part of the digital 3D model based on the fluorescence information, wherein the dental plaque parameter represents pathogenic plaque present on teeth of the dental situation, wherein detecting the dental plaque parameter comprises computing a function of the red (R) fluorescence signal value and the green (G) fluorescence signal value for the at least one unit element of the at least part of the digital 3D model,
- displaying the at least part of the digital 3D model and the detected dental plaque parameter.

Detecting the dental plaque parameter on the at least part of the digital 3D model based on the fluorescence information may comprise detecting the dental plaque parameter for the at least one unit element of the at least part of the digital 3D model. The dental plaque parameter may thus be detected per vertex, facet, point, node, voxel and/or pixel of the at least part of the digital 3D model, depending on the format of representation of the at least part of the digital 3D model.

Detecting the dental plaque parameter on the at least part of the digital 3D model may comprise comparing the green (G) fluorescence signal value of the at least one unit element of the at least part of the digital 3D model to a threshold value for green (G) fluorescence.

The threshold value for green (G) fluorescence may be a healthy reference value for green fluorescence (Gh). This healthy reference value characterizes healthy tooth tissue without pathogenic plaque presence.

The healthy reference value for green fluorescence (Gh) may be computed per each tooth of the digital 3D model. The healthy reference value for green fluorescence (Gh), for a tooth of the digital 3D model, may be obtained by sampling and averaging all values of green (G) fluorescence for all vertices belonging to that specific tooth.

Alternatively or additionally, the healthy reference value for green fluorescence (Gh), for the tooth of the digital 3D model, may be obtained by averaging all values of green (G) fluorescence for vertices belonging to the tooth, wherein the values of green (G) fluorescence are higher than a threshold value Gt. This additional condition may be advantageous in order to exclude regions with caries, in addition to regions comprising pathogenic plaque, from the computation of the healthy reference. Namely, caries may also emit fluorescence when illuminated. However, fluorescence profile of caries is characterized by loss in green (G) fluorescence value, while pathogenic dental plaque is characterized by the rise in the red (R) fluorescence value. By including only the values of green (G) fluorescence higher than the threshold value Gt in the computation for the healthy reference value for green fluorescence (Gh), it may be possible to obtain an accurate value for the healthy reference for green fluorescence (Gh) which may exclude presence of caries and/or pathogenic plaque. The threshold value Gt may also act as a filter to exclude nontooth material, such as fillings, that does not emit fluorescence when excited.

The healthy reference value for green fluorescence (Gh) may be obtained experimentally and be set as a predefined numerical value.

Detecting the dental plaque parameter, such as the pathogenic plaque presence value, on the at least part of the digital 3D model may comprise computing a difference of the green (G) fluorescence signal value of the at least one unit element of the at least part of the digital 3D model and the healthy reference value for green fluorescence (Gh). This difference may be expressed as G-Gh. Instead of, or in addition to the difference G-Gh, a ratio G/Gh may be utilized to detect pathogenic plaque presence value.

Detecting the dental plaque parameter, such as the pathogenic plaque presence value, on the at least part of the digital 3D model may comprise computing a function of the red (R) fluorescence signal value and the green (G) fluorescence signal value for the at least one unit element of the at least part of the digital 3D model. Overall, a more accurate result in pathogenic plaque detection may be obtained by evaluating, besides the green (G) fluorescence signal value, also the red (R) fluorescence signal value because it may be affected due to pathogenic plaque presence.

In one example, computing the function of the red (R) fluorescence signal value and the green (G) fluorescence signal value may comprise determining a difference signal by computing a difference between the red (R) fluorescence signal value and the green (G) fluorescence signal value for the at least one unit element of the at least part of the digital 3D model. Thus, the function in one embodiment may be the difference between the red (R) fluorescence signal value and the green (G) fluorescence signal value.

The difference between the red (R) fluorescence signal value and the green (G) fluorescence signal value, which may also be referred to as a difference signal, may be a particularly advantageous function because this difference signal has low noise contribution and a simple direct scaling with the desired red fluorescence signal, as will be demonstrated later.

Detecting the dental plaque parameter, such as the pathogenic plaque presence value, may comprise determining that an amplitude of the difference signal is larger than a noise component of the difference signal. The noise component of the difference signal may refer to noise contributions from both the red (R) fluorescence signal value and the green (G) fluorescence signal value.

In another example, the function of the red (R) fluorescence signal value and the green (G) fluorescence signal value for the at least one unit element of the at least part of the digital 3D model may be expressed as: $R-G-(R_h-G_h)$, wherein $R_h$ is a healthy reference value for red fluorescence and $G_h$ is the healthy reference value for green fluorescence. Both the healthy reference value for red fluorescence ($R_h$) and the healthy reference value for green fluorescence ($G_h$) may be computed per tooth of the digital 3D model.

The healthy reference value for red fluorescence ($R_h$) may be calculated in a similar way as previously described for the healthy reference value for green fluorescence ($G_h$). Namely, the healthy reference value for red fluorescence ($R_h$) may be computed per each tooth of the digital 3D model. The healthy reference value for red fluorescence ($R_h$), for a single tooth of the digital 3D model, may be obtained by sampling and averaging all values of red (R) fluorescence for all vertices belonging to that single tooth. Alternatively, the healthy reference value for red fluorescence ($R_h$), for the single tooth of the digital 3D model, may be obtained by sampling and averaging all values of red (R) fluorescence for all vertices used for calculating the healthy reference value for green fluorescence ($G_h$).

The healthy reference value for red fluorescence ($R_h$) may alternatively be obtained experimentally and be set as a predefined numerical value.

The utilization of function $R-G-(R_h-G_h)$ is advantageous as it accurately reflects the fluorescence behavior. Contribution of the term comprising healthy references is reflected in that it allows for normalization of different environmental and/or patient-characteristic conditions. Due to its sensitivity it allows for displaying the dental plaque parameter over large area of the teeth.

In another example, the function of the red (R) fluorescence signal value and the green (G) fluorescence signal value for the at least one unit element of the at least part of the digital 3D model may be a ratio of the red (R) fluorescence signal value and the green (G) fluorescence signal value, expressed as: $R/G$.

In yet a further example, the function of the red (R) fluorescence signal value and the green (G) fluorescence signal value for the at least one unit element of the at least part of the digital 3D model may be a difference of the ratio $R/G$ of the red (R) fluorescence signal value and the green (G) fluorescence signal value, and a ratio $R_h/G_h$ of the healthy reference value for red fluorescence ($R_h$) and the healthy reference value for green fluorescence ($G_h$). This difference of two ratios may be expressed as: $R/G-R_h/G_h$. The characteristic of this difference function of two ratios is that it may reflect a more realistic pathogenic plaque formation due to sharper edges visible on the digital 3D model. To dental practitioners, results obtained and visualized in this way provide a common and familiar view on pathogenic plaque formation.

The above examples of functions utilizing healthy references, $R-G-(R_h-G_h)$ and $R/G-R_h/G_h$, may be advantageous as they may provide insight into a relative increase of red fluorescence compared to green fluorescence, which is a characteristic of pathogenic plaque fluorescence profile. Due to this property, it may be possible to use these functions on multiple different 3D models for various dental situations, and obtain normalized results. Namely, these functions are robust to variable absolute values of green fluorescence in different people. Additionally, the fact that different 3D models are affected by different ambient light conditions during scanning is not reflected in obtained results. In general, these functions are less susceptible to factors affecting variability of the red (R) fluorescence signal value and the green (G) fluorescence signal value in different 3D models. The contribution of the terms comprising healthy references is reflected in that they allow for normalization of different environmental and/or patient-characteristic conditions.

The fluorescence information may, in an embodiment, be converted from values in the Red, Green, Blue (RGB) color space into values of the Lightness, Chroma and Hue (LCH) color space. Thus, the method according to an embodiment of the disclosure may further comprise, for each unit element of the at least part of the digital 3D model:

- converting the red (R) fluorescence signal value and the green (G) fluorescence signal value into Lightness, Chroma and Hue (LCH) values of Lightness, Chroma and Hue (LCH) color space,
- identifying a green color shade and/or a red color shade by setting a set of thresholds for Hue and/or Chroma of LCH color space,

- identifying the dental plaque parameter based on values of Hue and/or Chroma of LCH color space. The dental plaque parameter may particularly refer to the pathogenic plaque presence.

It may be possible to define a threshold value for Hue, Ht, such that pathogenic plaque is detected based on comparison of Hue components of converted fluorescence signal values to this Ht threshold. An example of the comparison may be a ratio H/Ht.

In an example, the threshold value Ht may be determined by converting the healthy reference value for red fluorescence (Rh) and the healthy reference value for green fluorescence (Gh) into Lightness, Chroma and Hue (LCH) color space.

In some examples, the method may utilize natural color information in addition to the fluorescence information. Detecting the dental plaque parameter, such as the pathogenic plaque presence value, on the at least part of the digital 3D model may comprise computing a function of the red (R) fluorescence signal value, the green (G) fluorescence signal value, and Red, Green, Blue (RGB) natural color values for the at least one unit element of the at least part of the digital 3D model.

The function utilizing both fluorescence information and natural color information may be optimized with a regression method based on human annotations of where the pathogenic plaque is present. Alternatively, the function utilizing both fluorescence information and natural color information may be optimized with a deep learning method based on human annotations of where the pathogenic plaque is present.

The method according to the disclosure may further comprise determining a plaque severity level value for the at least one unit element of the at least part of the digital 3D model as 1-G, wherein G is the green (G) fluorescence signal value corresponding to the at least one unit element of the at least part of the digital 3D model.

The plaque severity level may usually be calculated per tooth and/or per tooth surface. For that purpose, an average value of the plaque severity level values for a plurality of unit elements of the at least part of the digital 3D model may be determined.

The method according to the disclosure may comprise displaying, for example on the display screen, the at least part of the digital 3D model and the detected dental plaque parameter, such as the pathogenic plaque presence value.

Displaying may comprise coloring the at least one unit element of the at least part of the digital 3D model for which presence of the dental plaque parameter has been confirmed. In that manner, the respective unit elements such as facets on the digital 3D model in mesh form, may be colored in order to be easily distinguished from healthy regions of the digital 3D model.

Displaying of the at least part of the digital 3D model and the detected dental plaque parameter may comprise applying a color to the at least one unit element of the at least part of the digital 3D model as a function of the determined plaque severity level values. In this way, the user may be able to efficiently identify regions in the dental situation with the highest severity of plaque. The lower the value for the plaque severity level, the more intense the pathogenic plaque will look like, when displayed on the at least part of the digital 3D model.

As a result of the displaying step, the information about pathogenic plaque in the dental situation may be conveyed to the user. For example, the at least part of the digital 3D model comprising plaque may be highlighted to visually distinguish it from the rest of the digital 3D model. Additionally or alternatively, the at least part of the digital 3D model may be colored in a different color, or it may be encircled to visually distinguish the at least part of the digital 3D model comprising plaque from the rest of the digital 3D model. Alternatively, a line defining a border between the at least part of the digital 3D model comprising plaque and the rest of the digital 3D model may be highlighted.

In an example, displaying step may comprise displaying a tabular overview showing each tooth surface of the at least part of the digital 3D model and the corresponding dental plaque parameter. This tabular overview may be in the form of a dental chart. The dental chart may therefore be automatically populated with relevant information, bringing efficiency and time saving for the users who may be used to manually populate dental charts. Alternatively or additionally, the dental plaque parameter may be displayed directly on the digital 3D model, for example adjacent to corresponding tooth surface.

One particularly advantageous way of presenting plaque on the digital 3D model may be coloring the at least one unit element of the at least part of the digital 3D model, for which presence of the dental plaque parameter has been established, in a color corresponding to the color of a disclosing agent. For users such as dental practitioners this may be advantageous, as the presented results resemble use of the disclosing agent. A further advantage is that all patients may be given full oral hygiene assessment with respect to dental plaque, and not just patients deemed being "high-risk", because in dental offices, the disclosing agent is currently used only on "high-risk" patients.

Accurate displaying of the at least part of the digital 3D model and the detected dental plaque parameter may be particularly advantageous as it provides the user and the patient with an easy visual indication for where to brush teeth better. Another important advantage of the displaying is that patient's acceptance to dental assessments is improved because no physical disclosing agent needs to be used which may cause staining on clothes and requires removal. Thus, the complete method provided by the disclosure is purely a digital one.

One further advantage of the method according to the disclosure is that users such as dental hygienists may be able to use the method before a professional removal of plaque is performed. Afterwards, evaluation of quality of the removal may be performed and the results of evaluation may be documented and presented.

The method according to an embodiment may further comprise determining plaque density for the at least part of the digital 3D model. The plaque density may serve as another clinically relevant dental plaque parameter as it may give insight into the concentration level of plaque-causing bacteria present on teeth. Generally, the higher the concentration of bacteria present, the higher the plaque density and the risk for cariogenic and periodontal diseases.

In an example, determining plaque density may comprise, for at least one tooth surface of the tooth comprising the at least one unit element with the detected dental plaque parameter, counting a number of neighboring unit elements also comprising detected dental plaque parameter. The counting may be performed within a sphere of a pre-determined radius. In another example, determining plaque density may comprise computing a distance from each unit element with the detected dental plaque parameter to a closest unit element with the detected dental plaque parameter. Both examples provide valuable input into how dense the plaque is within the at least part of the digital 3D model.

The method may further comprise, based on the determined plaque density, coloring the at least one unit element of the at least part of the digital 3D model according to a density-based gradient color scheme. For example, regions with higher plaque density may be colored in darker colors while regions with low plaque density may be colored with brighter colors.

In an embodiment, the method may comprise obtaining a segmented 3D model by segmenting the at least part of the digital 3D model into dental objects such as teeth and gingiva. The segmented 3D model may comprise the at least one unit element of the at least part of the digital 3D model.

Segmenting the at least part of the digital 3D model may be performed via a segmentation process which allows for identification of distinct dental objects such as individual teeth and/or surrounding gingiva in the digital 3D model. Individual teeth can be assigned a tooth identifier, for example according to the Universal Numbering Notation (UNN) in which numerals 1-32 are assigned to human teeth. The segmentation process may comprise use of algorithms such as Principal Component Analysis (PCA) or harmonic fields. The segmentation process may alternatively or additionally comprise use of machine learning models.

Information obtained from the segmentation process may be used for coloring the correct unit element of the at least part of the digital 3D model for which presence of the dental plaque parameter has been established. In turn, this may enable better communication of the results of dental plaque parameter determination to the user.

In an embodiment, the method may further comprise, within the at least part of the digital 3D model comprising the dental plaque parameter, determining a first surface area which comprises plaque and/or pathogenic plaque, determining a second surface area which corresponds to plaque and/or pathogenic plaque absence, and determining a ratio of the first surface area to the sum of the first and second surface area. This ratio may be referred to as Planimetric Plaque Index (PPI) give insight into what percentage of the tooth surface comprises plaque and/or pathogenic plaque. This ratio may also be determined for the complete digital 3D model, thus giving insight into what percentage of overall teeth surface may be covered in plaque and/or pathogenic plaque.

According to an embodiment, the method may further comprise determining a first number of tooth surfaces comprising the dental plaque parameter, determining a second number of tooth surfaces without the dental plaque parameter, and determining a ratio of the first number of tooth surfaces to a sum of the first and second number of tooth surfaces. In this way a number of tooth surfaces with dental plaque parameter relative to the total number of examined surfaces may be obtained.

Tooth surfaces comprising the dental plaque parameter may be understood as surfaces comprising plaque and/or pathogenic plaque. Tooth surfaces without the dental plaque parameter may be understood as healthy tooth surfaces, with plaque and/or pathogenic plaque absent.

This ratio of the first number of tooth surfaces to a sum of the first and second number of tooth surfaces may be referred to as plaque index if the dental plaque parameter is plaque presence value, and may provide insight into what percentage of overall tooth surfaces may be covered in plaque. Alternatively, the above ratio may be referred to as pathogenic plaque index if the dental plaque parameter is pathogenic plaque presence value and may provide insight into what percentage of overall tooth surfaces may be covered in pathogenic plaque. The plaque index determined according to the method of the disclosure is more accurate, and reliable than determining the plaque index by manually counting tooth surfaces which the dental practitioner may consider relevant.

According to an embodiment, the plaque index may be calculated by assigning a score to each tooth surface of the tooth and obtaining a total score for the tooth by dividing a sum of scores for each tooth surface with a number of tooth surfaces of the tooth. The score assigned to each tooth surface may be a numerical value between 0 and 3. Additionally or alternatively, the score assigned to each tooth surface may be a numerical value between 0 and 5 in which the total score corresponds to clinical measure known as the Turesky modified Quigley Hein Plaque Index (TQHPI). The score assigned to each tooth surface may correspond to plaque density of the each tooth surface. Alternatively, the score assigned to each tooth surface may correspond to plaque thickness for the each tooth surface.

The obtained dental plaque parameters, plaque density and/or plaque index measures may be part of overall patient's dental health score calculation.

In one embodiment of the disclosure, the method may further comprise:

- receiving, by the processor, at least a part of a further digital 3D model of the dental situation, wherein the at least part of the further digital 3D model may comprise further fluorescence information associated with fluorescence emitted from dental plaque present on teeth of the dental situation, the further digital 3D model representing the dental situation captured by the intraoral scanner at a later time point compared to the digital 3D model,

- detecting a further dental plaque parameter on the at least part of the further digital 3D model based on the further fluorescence information, wherein the further dental plaque parameter represents pathogenic plaque present on teeth of the dental situation at the later time point,

- displaying the at least part of the further digital 3D model and the detected further dental plaque parameter.

As for the digital 3D model, a further dental plaque parameter may be determined for the further digital 3D model based on the further fluorescence information.

The at least part of the digital 3D model and the at least part of the further digital 3D model may be geometrically aligned. Geometrically aligned the at least part of the digital 3D model and the further digital 3D model may also be referred to as a superimposed 3D model. In order to geometrically align the two digital 3D models, the at least part of the further digital 3D model may be segmented.

The method according to an embodiment may comprise obtaining a further segmented 3D model by segmenting the at least part of the further digital 3D model into a further plurality of teeth and gingiva. The further segmented 3D model may comprise the at least one unit element of the at least part of the further digital 3D model. Segmentation may be performed according to the segmentation process described previously.

By having available two digital 3D models of the same dental situation, differences between the dental plaque parameters of the two digital 3D models may be observed.

The superimposed 3D model may be displayed on the display screen. A controller may be arranged which may allow for adjustable display of the dental plaque parameter and/or the further dental plaque parameter on the superimposed digital 3D model. By means of user manipulation, the controller may be positioned between a first and a second position. The first position of the controller may correspond to displaying substantially only the dental plaque parameter on the superimposed digital 3D model. The second position of the controller may correspond to displaying substantially only the further dental plaque parameter on the superimposed digital 3D model. In this manner, a visual representation of plaque progress in the dental situation may be obtained. There may be further positions of the controller between the first and second position that may correspond to displaying at least a portion of both the dental plaque parameter and the further dental plaque parameter. The controller may be referred to as a slider or a 3D slider.

In further examples, displaying of the at least part of the digital 3D model and the at least part of the further digital 3D model may be performed through simulation or morphing, to visualize time progression of the detected dental plaque parameter.

A computer program product is further disclosed. The computer program product according to an embodiment may comprise instructions which, when the program is executed by a computer, causes the computer to carry out the method of any one or more of presented embodiments.

Further, the disclosure may comprise a non-transitory computer readable medium. The non-transitory computer readable medium may comprise instructions which, when executed by the computer, cause the computer to carry out the method of any one or more of presented embodiments.

**Brief description of the figures**

Aspects of the disclosure may be best understood from the following detailed description taken in conjunction with the accompanying figures. The figures are schematic and simplified for clarity, and they just show details to improve the understanding of the claims, while other details are left out. The individual features of each aspect may each be combined with any or all features of other aspects. These and other aspects, features and/or technical effects will be apparent from and elucidated with a reference to the illustrations described hereinafter in which:

Figure 1 illustrates a user interface with a displayed digital 3D model of a dental situation.

Figure 2 is a flow chart illustrating a method according to an embodiment.

Figure 3A shows possible visualization of pathogenic plaque determined by utilizing a function of red (R) fluorescence signal values and green (G) fluorescence signal values.

Figure 3B shows possible visualization of pathogenic plaque determined by utilizing a further function of red (R) fluorescence signal values and green (G) fluorescence signal values.

Figure 4 illustrates a user interface for detecting and visualizing plaque on the digital 3D model of the dental situation.

Figure 5A shows a digital 3D model with a displayed dental plaque parameter and a plaque index.

Figure 5B shows a superimposed digital 3D model and a further digital 3D model with displayed dental plaque parameter and the plaque index.

Figure 6 illustrates a trained neural network used in a method according to an embodiment.

Figure 7 is a flowchart illustrating a method to train a neural network for plaque detection.

Figure 8 illustrates a dental scanning system.

Figure 9 illustrates an exemplary workflow of a patient's visit to a dental practitioner.

**Detailed description**

In the following description, reference is made to the accompanying figures, which show by way of illustration how the invention may be practiced.

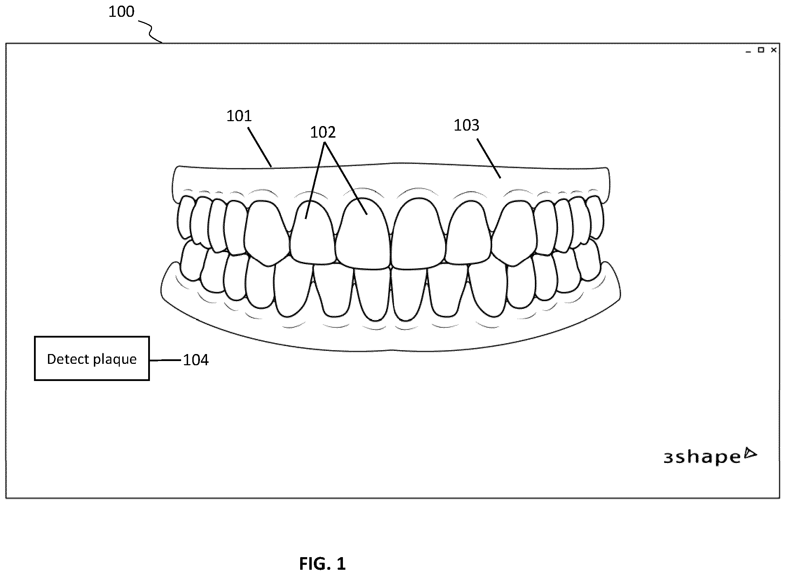

Figure 1 illustrates a user interface 100 with a displayed digital 3D model 101 of a patient's dental situation. The digital 3D model 101 may be displayed on a display screen in form of a 3D mesh, a point cloud, a 3D graph, a volumetric representation, or any other suitable 3D representation form.

The digital 3D model 101 may comprise a lower jaw and/or an upper jaw with multiple teeth representations. The digital 3D model 101 accurately represents the patient's dental situation and may be constructed based on scan data collected in a scanning process in which, for example an intraoral scanner 825 is used to scan the patient's dental situation comprising teeth and gingiva. The captured scan data can be used to construct the digital 3D model 101 and render it on the display screen. The scan data or the digital 3D model 101 can be stored in and accessed from a memory device of a computer system. The collected scan data may comprise natural color information associated with the natural color of teeth and/or gingiva, geometric information associated with placement of dental objects in 3D space as well as infrared information and/or fluorescence information related to the patient's dental situation.

The user interface 100 may comprise a button 104 which, once engaged by a user, may initiate the method 200 for detecting dental plaque on the digital 3D model 101 according to any embodiment of the disclosure. Throughout this disclosure, term "plaque" may be used to refer to dental plaque. Detecting plaque may comprise identifying presence of plaque and/or any other dental plaque parameter such as plaque thickness and/or plaque type. The most relevant plaque type to detect, for diagnostic purposes, is pathogenic plaque because it may lead to cariogenic or periodontic diseases if untreated. The method according to the disclosure may also be initiated automatically, without user engagement.

To identify and separate individual teeth 102 and gingiva 103 within the digital 3D model 101, the digital 3D model 101 may be segmented. This means that unit elements, for example facets of the 3D mesh representation of the digital 3D model 101, belonging to individual teeth 102 as per Universal Numbering System/Notation (UNN), may be determined. In this way individual teeth 102, or the unit elements of the individual teeth 102, may be analyzed for plaque presence. Segmentation of the digital 3D model 101 is an optional step, and the complete digital 3D model 101 may instead be analyzed for identifying plaque.

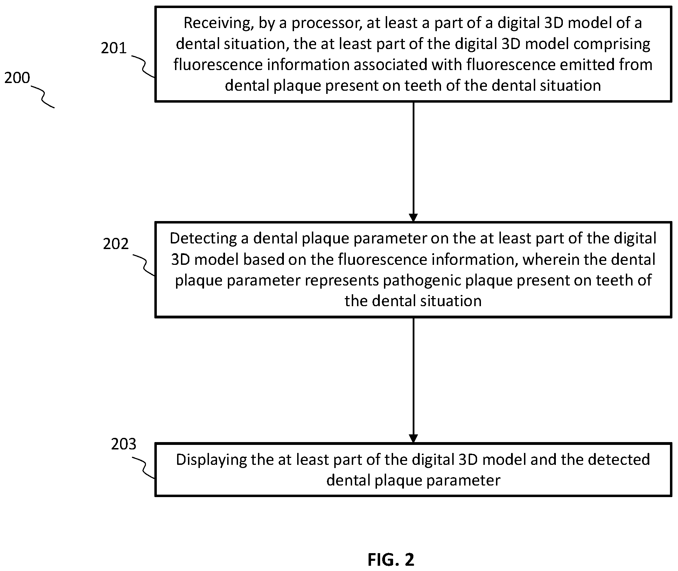

Figure 2 shows a flowchart illustrating a method 200 according to an embodiment.

First, at least a part of the digital 3D model 101 may be received, by the processor, in step 201. The at least part of the digital 3D model 101 may comprise fluorescence information associated with fluorescence emitted from dental plaque present on teeth of the dental situation.

The at least part of the digital 3D model 101 may be stored in a memory of a computer system, for example in a Standard Triangle Language (STL) format. The digital 3D model 101 may be received by the processor, for example when the user engages the button 104 in the user interface 100. The at least part of the digital 3D model 101 may usually be displayed on the display screen in the form of the 3D mesh, the point cloud, the 3D graph, the volumetric representation, or any other suitable 3D representation form.

Fluorescence information may comprise a red (R) fluorescence signal value and a green (G) fluorescence signal value for at least one unit element of the at least part of the digital 3D model 101. The fluorescence information may therefore be represented in numerical form in Red, Green, Blue (RGB) color space.

The fluorescence information may be recorded by means of the intraoral scanner 825 equipped with a blue light source capable of exciting fluorescent material in the dental situation, an image sensor capable of recording the emitted fluorescence, and a filter to block the excitation light from the sensor while transmitting the fluorescent light. The pathogenic plaque, present on teeth of the dental situation, is such fluorescent material which emits fluorescence when illuminated with the blue light source with wavelength of around 400 nanometers, for example, 405 nanometers. The emitted fluorescence can then be observed.

The red fluorescence, indicating bacteria present in pathogenic plaque, may thus be detected and relevant information may be deduced from the detected fluorescence. A blue (B) fluorescence signal value may be filtered out during the scanning process or afterwards and its use is not necessary. The blue (B) fluorescence signal value may therefore be zero.

Step 202 of the method 200 illustrates detecting a dental plaque parameter 301 on the at least part of the digital 3D model 101 based on the fluorescence information, wherein the dental plaque parameter 301 represents pathogenic plaque present on teeth of the dental situation. The dental plaque parameter 301 may preferably refer to presence of pathogenic plaque, which is old plaque and of a particular significance due to cariogenic and periodontic properties.

In an embodiment, the dental plaque parameter 301 may be a plaque presence value. By deriving information on pathogenic plaque presence, information on plaque presence becomes available. For some use cases, for example calculation of plaque index, simple information on plaque presence, for example in form of "YES" and "NO" values, may be required.

Step 203 of the method 200 illustrates displaying the at least part of the digital 3D model 101 and the detected dental plaque parameter 301. Displaying may occur for example on the display screen such as that of a computer, a tablet, a phone or any similar device.

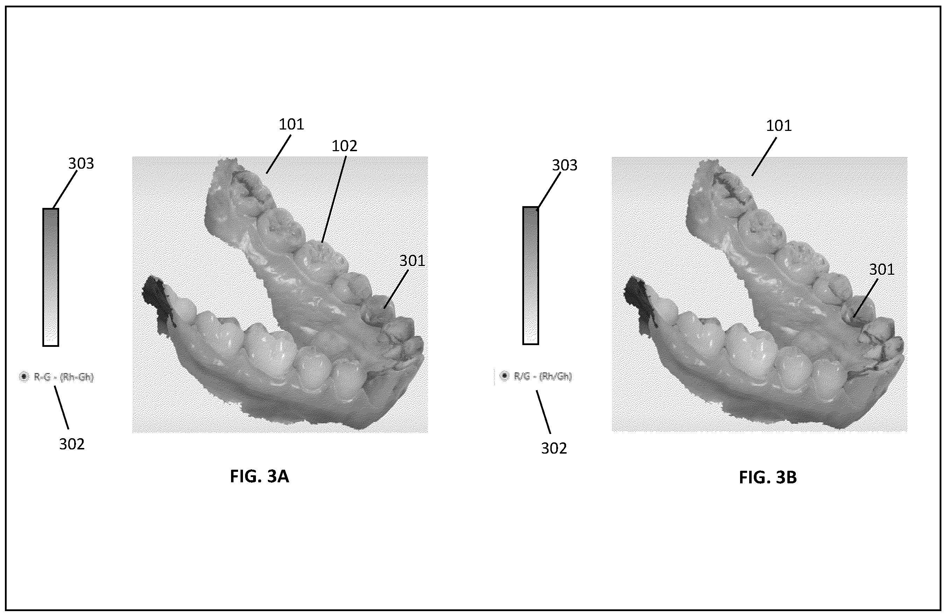

Displaying may comprise displaying the at least part of the digital 3D model 101 with highlighted unit elements, for example facets, for which the dental plaque parameter 301 has been detected. Displaying of the at least part of the digital 3D model 101 may be further understood with reference to figures 3, 4 and 5.

The dental plaque parameter 301 may be detected for at least one unit element of the at least part of the digital 3D model 101. The at least one input element may be a facet and/or a vertex of the 3D mesh, a point of the point cloud, a voxel of the volumetric representation or a pixel of the 2D image. In general, the at least one unit element of the at least part of the digital 3D model 101 may be a pixel, a voxel, a vertex, a facet, a node and/or a point, depending on the representation format of the at least part of the digital 3D model 101.

Detecting the dental plaque parameter 301 on the at least part of the digital 3D model 101 may comprise computing a function of the red (R) fluorescence signal value and the green (G) fluorescence signal value for the at least one unit element of the at least part of the digital 3D model 101. Several different functions have been proven to be effective for detecting the dental plaque parameter 301 such as presence of pathogenic plaque.

For example, the function may be a ratio R/G of the red (R) fluorescence signal value and the green (G) fluorescence signal value for the at least one unit element of the at least part of the digital 3D model 101.

Another example of the function may be a difference R-G of the red (R) fluorescence signal value and the green (G) fluorescence signal value for the at least one unit element of the at least part of the digital 3D model 101.

These two examples of functions are further discussed in the following section.

Healthy tooth tissue, without presence of any plaque, emits green autofluorescence $G_{BG}$ and no red fluorescence. Teeth regions with plaque of level $s$ emit red fluorescence $\Delta R(s)$, and possibly a small added value of green fluorescence $\Delta G(s)$.

The respective fluorescence signals in the green (G) and red (R) channels can then be expressed as follows:

$$G(s) = G_{BG} + \Delta G(s) \approx G_{BG} + \Delta G \cdot s, \qquad (1)$$

$$R(s) = \Delta R(s) \approx R_{BG} + \Delta R \cdot s \qquad (2)$$

In the above equations, it may be assumed that the fluorescence signals $\Delta R(s)$ and $\Delta G(s)$ scale roughly linear with the contributing plaque level s.

The green autofluorescence signal of the healthy tooth tissue may be quite strong and may give a much larger signal than any added green fluorescence signal $\Delta G(s)$ from plaque. The effect of any presence of plaque on the green fluorescence might even be a reduction in the signal, since a thick plaque layer might obscure the green autofluorescence from the tissue behind the plaque. Thus, the value for green fluorescence signal from plaque $\Delta G$ might be either positive or negative, but remains much smaller in size than the green autofluorescence $G_{BG}$.

A more realistic representation of the fluorescence signals in the green (G) and red (R) channels can be expressed as:

$$G(s) = B_{BG} + G_{BG} + \Delta G(s) + N_G \qquad (3)$$

$$R(s) = B_{BG} + \Delta R(s) + N_B, \qquad (4)$$

where $B_{BG}$ is the background signal from the blue transmitted light assumed equal in the red and green channels. $N_G$ and $N_B$ are the noise contributions in the green and red channel, respectively. These noise contributions are inherently random in nature and therefore different for the two channels, even though they are similar in magnitude.

Equations (3) and (4) may be explained by considering the intraoral scanner 825 as a device with limited size and heat management, as well as with limitation on optical power due to hazard and safety regulations. Additionally, the images obtained through scanning may contain noise to a certain degree due to the limited size of a detector in the intraoral scanner 825 and lack of efficient cooling. Even when appropriate filters are used to block the blue excitation light, some blue light may still be transmitted to the image sensor and consequently add a background signal to the green and red channels by electron creep phenomenon.

The ratio R/G of the red (R) fluorescence signal value and the green (G) fluorescence signal value may be represented as:

$$\frac{R(s)}{G(s)} = \frac{B_{BG} + \Delta R(s) + N_B}{B_{BG} + G_{BG} + \Delta G(s) + N_G} \approx \frac{B_{BG} + \Delta R \cdot s + N_B}{B_{BG} + G_{BG} + N_G}$$

$$\approx BG + \frac{\Delta R \cdot s + N_B}{\overline{BG} + N_G}, \qquad (5)$$

where the green contribution from plaque $\Delta G(s)$ is assumed much smaller than the combined contribution from autofluorescence and blue background signal, so that following applies:

$$(B_{BG} + G_{BG} + \Delta G(s) \approx B_{BG} + G_{BG}) \qquad (6)$$

The background signals may be collected into single symbols, i.e.

$$BG = B_{BG}/(B_{BG} + G_{BG} + N_G) ) \qquad (7),$$

and

$$\overline{BG} = B_{BG} + G_{BG} \qquad (8)$$

Assuming $BG > N_G$, a Taylor series expansion of ratio $R(S)/G(S)$ to second orders order yields:

$$\frac{R(s)}{G(s)} = BG + \frac{\Delta R \cdot s + N_B}{\overline{BG} + N_G} \approx BG + (\Delta R \cdot s + N_B)\left(\frac{1}{\overline{BG}} - \frac{N_G}{\overline{BG}^2}\right)$$

$$= BG + \frac{(\Delta R \cdot s + N_B)}{\overline{BG}}\left(1 - \frac{N_G}{\overline{BG}}\right) \qquad (9)$$

The rightmost term in equation (9) comprising noise term $N_G$ contributes to the noise without contributing to the signal. A signal-to-noise ratio is therefore reduced compared to a situation where the noise term $N_G$ is absent.

Thus, the ratio R/G may be characterized with unwanted noise.

The function in form of the difference R-G may result in improvements in signal-to-noise ratio:

$$R(s) - G(s) = B_{BG} + \Delta R(s) + N_R - (B_{BG} + G_{BG} + \Delta G(s) + N_G)$$

$$\approx -G_{BG} + \Delta R \cdot s + (N_R - N_G)$$

$$= \Delta R \cdot s + \hat{N} - G_{BG}, \qquad (10)$$

where $\hat{N} = (N_B - N_G)$ is a common noise for both channels with same magnitude as each noise contribution.

For the difference signal expressed in equation (10), the blue background signal $\hat{B}_{BG}$ is removed completely, and the difference R-G scales directly with the desired red fluorescence signal $\Delta R \cdot s$.

It can be observed that, given the noise present in a system of restricted size such as the intraoral scanner 825, the ratio R/G may provide a result with higher noise content than the difference R-G.

In another example, the function of the red (R) fluorescence signal value and the green (G) fluorescence signal value for the at least one unit element of the at least part of the digital 3D model 101 may be expressed as: R-G-(Rh-Gh), wherein Rh is a healthy reference value for red fluorescence and Gh is a healthy reference value for green fluorescence. Both the healthy reference value for red fluorescence (Rh) and the healthy reference value for green fluorescence (Gh) may be computed per tooth 102 of the digital 3D model 101.

The healthy reference value for green fluorescence (Gh), for a tooth 102 of the digital 3D model 101, may be obtained by sampling and averaging all values of green (G) fluorescence for all unit elements, for example vertices, comprised in the tooth 102.

Alternatively, the healthy reference value for green fluorescence (Gh), for the tooth 102 of the digital 3D model 101, may be obtained by averaging all values of green (G) fluorescence higher than a threshold value Gt, for all unit elements, for example vertices, belonging to the tooth 102. This additional condition may be advantageous in order to exclude detection of caries and/or pathogenic plaque from healthy reference calculation.

Namely, caries may also emit fluorescence when illuminated with the blue light used to detect dental plaque parameters. However, caries may be characterized by loss in green (G) fluorescence value, while pathogenic dental plaque may be characterized by the rise in the red (R) fluorescence value. By including only the values of green (G) fluorescence higher than the threshold value Gt in the computation for the healthy reference value for green fluorescence (Gh), it may be possible to obtain a correct value for the healthy reference by excluding false-positives in form of caries and/or pathogenic plaque regions.

Alternatively, the healthy reference value for green fluorescence (Gh) may be obtained experimentally and be set as a predefined numerical value.

The healthy reference value for red fluorescence (Rh) may be calculated in a similar way as described for the healthy reference value for green fluorescence (Gh). Namely, the healthy reference value for red fluorescence (Rh) may be computed per tooth 102 of the digital 3D model 101. The healthy reference value for red fluorescence (Rh), for the tooth 102 of the digital 3D model 101, may be obtained by averaging all values of red (R) fluorescence for all unit elements, for example vertices, belonging to that single tooth 102. Alternatively, only unit elements, for example vertices, used for calculating the healthy reference value for green fluorescence (Gh) may be used in calculating the healthy reference value for red fluorescence (Rh).

The healthy reference value for red fluorescence (Rh) may alternatively be obtained experimentally and be set as a predefined numerical value.

The utilization of difference function R-G-(Rh-Gh) is advantageous as it accurately reflects fluorescence behavior. Contribution of the term comprising healthy references is reflected in that it allows for normalization of different environmental and/or patient-characteristic conditions. Due to its sensitivity it allows for displaying the dental plaque parameter 301 over large area of the teeth.

In yet a further example, the function of the red (R) fluorescence signal value and the green (G) fluorescence signal value for the at least one unit element of the at least part of the digital 3D model 101 may be a difference of the ratio R/G of the red (R) fluorescence signal value and the green (G) fluorescence signal value, and a ratio Rh/Gh of the healthy reference value for red fluorescence (Rh) and the healthy reference value for green fluorescence (Gh). This difference may be expressed as: R/G-Rh/Gh. The characteristic of this function of difference of two ratios is that it may reflect a more realistic pathogenic plaque formation due to sharper edges visible on the digital 3D model 101. To dental practitioners, results obtained and visualized in this way provide a common and familiar view on pathogenic plaque formation.

The utilization of functions with healthy references, R-G-(Rh-Gh) or R/G-(Rh/Gh), may be advantageous as they may provide insight into a relative increase of red fluorescence compared to green fluorescence, which is a characteristic of pathogenic plaque fluorescence profile. Due to this property, it may be possible to use these functions on multiple different 3D models for various dental situations and obtain normalized results. Namely, these functions are robust to variable absolute values of green fluorescence in different people. Additionally, the fact that different 3D models are affected by different ambient light conditions during scanning is not reflected in obtained results. In general, these functions are less susceptible to factors affecting variability of the red (R) fluorescence signal value and the green (G) fluorescence signal value in different 3D models. The contribution of the terms comprising healthy references is reflected in that they allow for normalization of different environmental and/or patient-characteristic conditions.

Figure 3A illustrates the digital 3D model 101 with displayed dental plaque parameter 301. The dental plaque parameter 301 may be pathogenic plaque presence value. The pathogenic plaque presence value, for the at least one unit element of the at least part of the digital 3D model 101, may be determined as the function 302 of the red (R) fluorescence signal value and the green (G) fluorescence signal value. In case of figure 3A, the function 302 used is: R-G-(Rh-Gh). For example, this function may be applied to each vertex on the digital 3D model 101 and according to the obtained value, the vertices on the digital 3D model may be colored. A gradient bar 303 may serve as a legend to indicate what shade of color is used to visualize results of the function 302. The darkest shade of color may indicate highest density of pathogenic plaque. The lightest shade may indicate lowest density of pathogenic plaque or absence of pathogenic plaque.

Figure 3B illustrates the digital 3D model 101 with displayed dental plaque parameter 301. In case of figure 3B, the function 302 used is: R/G-(Rh/Gh). For example, this function may be applied to each vertex on the digital 3D model 101 and according to the obtained value, the vertices on the digital 3D model may be colored. The gradient bar 303 may serve as a legend to indicate what shade of color is associated to display results of the function 302. The darkest shade of color may indicate the highest density of pathogenic plaque. The lightest shade may indicate lowest density of pathogenic plaque or absence of pathogenic plaque. Results obtained with the function 302 being R/G-(Rh/Gh) may accurately reflect the pathogenic plaque formation, and more pronounced edges of coloring can be observed on the digital 3D model 101.

Displaying of the at least part of the digital 3D model 101 and the detected dental plaque parameter 301 may comprise coloring of the at least one unit element of the at least part of the digital 3D model 101 according to a result of the function 302 used. The at least part of the digital 3D model 101 may be colored according to the gradient bar 303. Thereby, a darker shade of color may be used to visualize more dense, highly pathogenic plaque. Alternatively or additionally, the at least part of the digital 3D model 101 may be colored according to a plaque severity value, wherein the plaque severity value can be obtained as 1-G, wherein G is the green (G) fluorescence signal value for the at least one unit element of the at least part of the digital 3D model 101.

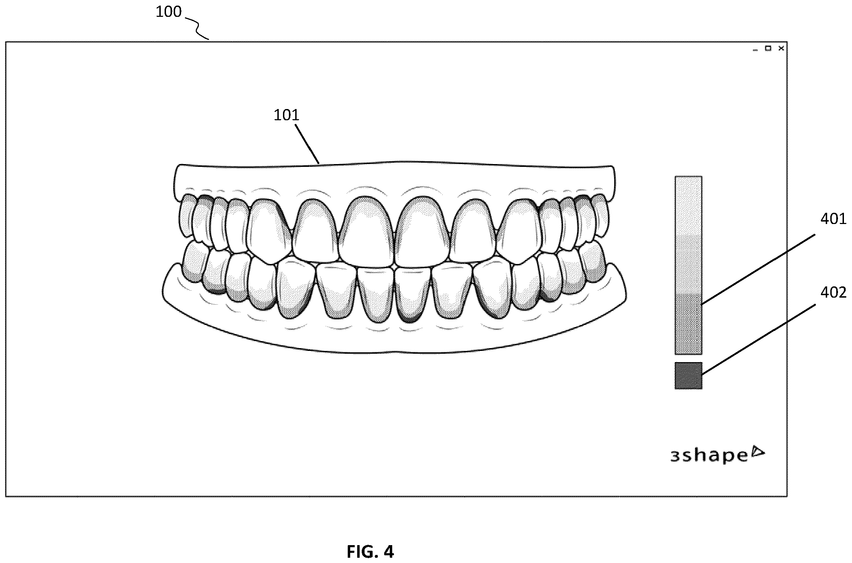

Figure 4 illustrates the user interface 100 for detecting and visualizing plaque on the digital 3D model 101 of the dental situation. The digital 3D model 101 may be colored according to the obtained dental plaque parameter 301 in the method 200 for determining plaque. Coloring may be performed per unit element of the digital 3D model 101, for example per vertex, facet, voxel, node and/or point. Colors used in visualization on figure 4 preferably correspond to colors of a disclosing agent used to stain plaque in the actual dental situation.

Figure 4 shows the digital 3D model 101 with visualized detected pathogenic plaque. An indicator 402 shows which color may be used to visualize the pathogenic plaque on teeth of the digital 3D model 101. In addition to visualizing pathogenic plaque, the virtual 3D model 101 may also visualize an additional dental plaque parameter, namely a plaque thickness value. Depending on the detected plaque thickness value, the unit elements of the digital 3D model 101 may be colored in a different color shade. This has effect of visually differentiating a thin, medium or thick plaque layers. A gradient bar 401 may be displayed and may serve as a legend to interpret how thick a specific point of plaque is. The plaque thickness value may also be displayed if the user brings the pointer, for example a mouse pointer, over a point on the digital 3D model 101 that comprises plaque.

The colors used to visualize pathogenic plaque may usually be darker compared to colors used to visualize thickness of plaque.

Thickness of dental plaque and/or plaque presence may be determined by use of a trained neural network 600, as described in following.

The method according to an embodiment of the disclosure may further comprise:

- providing the at least part of the digital 3D model 101 as an input 601 to a trained neural network 600, wherein the at least part of the digital 3D model 101 comprises natural color information and/or geometric information associated with the at least one tooth surface of the dental situation,
- obtaining an output 605 from the trained neural network 600 based on the provided input 601, wherein the output 605 comprises a plaque presence value and/or a plaque thickness value associated with the at least one tooth surface,
- assigning the plaque presence value and/or the plaque thickness value to the at least part of the digital 3D model 101.

The method may comprise displaying the digital 3D model 101 with the plaque presence value and/or the plaque thickness value assigned to the at least part of the digital 3D model 101.

The at least part of the digital 3D model 101 may correspond to the at least one tooth surface, or a part of the at least one tooth surface of the dental situation. The trained neural network 600 may be suitable for processing the at least part of the digital 3D model 101 directly in its three-dimensional format or in a two-dimensional representation of the at least part of the digital 3D model 101, such as a 2D image or a plurality of 2D images which may be generated by taking snapshots of the digital 3D model or which may be generated directly by the intraoral scanner.

In an embodiment, the input 601 to the trained neural network 600 may be in 3D format. An example of the input in 3D format may be the point cloud, the 3D mesh, the volumetric representation or the graph representation of the at least part of the digital 3D model 101. It may be possible to transform the input from one 3D representation type, for example from the mesh representation, into another 3D representation type, for example the point cloud representation. A type of the trained neural network 600 capable of processing the input in 3D format may be a trained PointNet network, or a PointNet-type network. These neural networks may be capable of processing the digital 3D model 101, or the at least part of the digital 3D model 101, in the point cloud form.

Providing the at least part of the digital 3D model 101 as the input 601 may comprise sampling the at least part of the digital 3D model 101 or converting the at least part of the digital 3D model 101 into a numerical form. For example, one or more points from the point cloud or mesh representation of the digital 3D model 101 may be converted into numerical form, for example a matrix of input values. The matrix may then form the input 601 for the trained neural network 600.

If the digital 3D model 101 is in the 3D mesh form, it may be possible to convert the digital 3D model 101 into the point cloud form, by collapsing all vertices from the 3D mesh format so that only points remain, thus forming the point cloud. The remaining point cloud may then be processed directly by the PointNet neural network, or the PointNet-type neural network.

In another embodiment, the input 601 may be in 2D format such as a 2D image or a plurality of 2D images of the at least one tooth surface of the digital 3D model 101.

The trained neural network 600 may thus be suitable for processing 2D input format. One or more of 2D images may be generated from the digital 3D model 101 by a virtual camera, referred to also as the camera. The virtual camera may take snapshots of the individual teeth of the digital 3D model 101.

For example, a tooth 102 in the digital 3D model 101 may be positioned such that a desired tooth surface of the tooth 102 is facing the camera. Positioning of the tooth 102 may comprise rotation in order to align the virtual camera direction and the target tooth surface. Positioning of the tooth 102 may also comprise translation such that the complete target tooth surface is in a field of view of the virtual camera, for example such that all facets on the tooth surface can be captured. Both rotation and translation may be performed via a local coordinate system of the tooth. This may be an automatic process where predetermined movements of the tooth 102 have been set up in order to capture, via the virtual camera, the one or more of the tooth surfaces. Alternatively, the virtual camera may be moved to a plurality of predetermined positions to capture snapshots of the desired tooth surface. The snapshots of the individual teeth may alternatively be generated by manual movement of the virtual camera to desired positions.

The method according to an embodiment may comprise generating the input 601 for the trained neural network 600, wherein the input 601 is the one or more 2D images of the at least one tooth surface of the tooth 102 in the digital 3D model 101, comprising:

- rotating the tooth 102, using the local coordinate system of the tooth, such that a surface normal of the at least one tooth surface of the tooth coincides with a direction of the virtual camera,
- translating the tooth 102, using the local coordinate system of the tooth, such that the at least one tooth surface is in the field of view of the virtual camera,
- creating the one or more 2D images by taking snapshots of the at least one tooth surface with the virtual camera.

Providing the at least part of the digital 3D model 101 as the input 601 for the trained neural network 600 may comprise generating one or more 2D images of the at least one tooth surface. The at least one tooth surface may be comprised in the digital 3D model 101 and may have a directly corresponding tooth surface in the actual dental situation. Thus, the process of generating the one or more 2D images of the at least one tooth surface of the digital 3D model 101 with a virtual camera may correspond to taking the photographs of the at least one tooth surface in the actual dental situation.

The generated one or more 2D images may form the input 601 for the trained neural network 600. Alternatively or additionally, the input 601 may be at least one pixel belonging to the one or more 2D images. The one or more 2D images may be generated by means of the virtual camera.

In an embodiment, providing the at least part of the digital 3D model 101 as the input 601 for the trained neural network 600 may comprise providing the 3D mesh of the at least part of the digital 3D model to the trained neural network 600 directly.

In a further embodiment, providing the at least part of the digital 3D model 101 as the input 601 for the trained neural network 600 may comprise providing the point cloud of the at least part of the digital 3D model 101 to the trained neural network 600 directly.

In yet a further embodiment, providing the at least part of the digital 3D model 101 as the input 601 for the trained neural network 600 may comprise providing the voxel representation of the at least part of the digital 3D model 101 to the trained neural network 600 directly.

The at least part of the digital 3D model 101 may comprise natural color information and/or geometric information associated with the at least one tooth surface of the dental situation. The tooth surface may be any one of an occlusal, a labial, a buccal, a mesial, a distal a cervical and/or an incisal surface.

Natural color information and/or geometric information may be comprised in the scan data obtained via the intraoral scanner 825 used to scan the dental situation.

Natural color information may relate to surface color of scanned teeth and/or gingiva and may be obtained by scanning the intraoral situation of the patient by means of the intraoral scanner 825. This information may be expressed through Red-Green-Blue (RGB) intensity values. The presence of natural color information may be beneficial as it may facilitate identifying plaque, because plaque may be of different color compared to uncovered enamel, for example it can be more yellow. The difference in color of tooth surfaces may therefore indicate plaque. Thus, the natural color information may be of high relevance for the trained neural network 600 in a process of detecting plaque presence, plaque type, but also stains, calculus and/or food debris.

Geometric information, also referred to as topological information, comprised in the at least part of the digital 3D model 101 may be depth information relating to a camera position, an angle between a facet normal and a camera direction, the facet normal and/or curvature information. The term "camera" in this context may relate to the virtual camera used to view the digital 3D model 101. The camera may be characterized by a virtual camera property, for example field of view (FOV) and/or focal length. Geometric information may be significant in detecting plaque as for example, plaque is characterized by different surface waviness compared to clean tooth surface without plaque.

The depth information may comprise distance information of the at least part of the digital 3D model 101 to the camera. Depth information may be in the form of a depth image. The depth image may display the at least part of the digital 3D model 101 according to its distance from the camera. For example, parts of the digital 3D model 101 more distant to the camera may be shaded darker than the parts closer to the camera. A distance threshold may be defined such that, when a distance between the camera and the part of the digital 3D model 101 is greater than the distance threshold, the part of the digital 3D model 101 is not shown in the depth information. The distance of the at least part of the digital 3D model 101 from a given camera position indirectly describes a topology of the at least part of the digital 3D model 101, and consequently a smoothness of a surface of the at least part of the digital 3D model 101. The variations of surface smoothness may represent a relevant parameter in detecting plaque in the at least part of the digital 3D model 101.

The facet normal may be a vector perpendicular to a facet of the digital 3D model 101. The facet can be seen as a unit element of the digital 3D model 101 in 3D mesh format and can, for example, be a triangle if the digital 3D model 101 is in format of a triangular mesh. A triangular facet may be defined by three points (vertices) interconnected with three edges. Two neighboring facets that have a small variation in their respective facet normals may indicate a local area that is smooth. However, a significant variation in the facet normals, if detected, may be an indication of plaque. The variation in two surface normals can be expressed in terms of an angle between the two surface normals.

The curvature information may be in the form of a per-facet value and may be obtained by measuring a change along facet normals of neighboring facets of the digital 3D model 101. This change may give insight into whether the associated section of the digital 3D model 101 is flat or curved. Thereby, the curvature information may describe topology of teeth by revealing smooth and non-smooth surfaces. Plaque causes changes at the surface level of teeth which then may be reflected directly in change of the surface curvature. Plaque may also be associated with a localized smoothness (regions of low curvature) in certain areas. The curvature information may thus be a significant parameter in detecting plaque presence, detecting locations of plaque, plaque type and/or plaque severity.

Optionally, the method may further comprise providing semantic information of the at least part of the digital 3D model 101 to the trained neural network 600. Thus, the input 601 may additionally comprise semantic information of the at least part of the digital 3D model 101.

Semantic information of the at least part of the digital 3D model 101 may be a tooth identifier, a tooth surface identifier, and/or a jaw identifier. The tooth surface identifier may provide information on which tooth surface is comprised in the input (e.g. a buccal, lingual, mesial, distal or occlusal surface) while the jaw identifier may provide information to what jaw (upper or lower) the at least part of the digital 3D model 101 relates to.

By incorporating semantic information into the input 601 of the trained neural network 600, a semantic context may be given to data present in the input, thereby providing semantic context to the trained neural network 600. The trained neural network 600 may consequently adapt its behavior according to the provided semantic information. Semantic information may be converted to numerical format, as other data formats, before being processed by the trained neural network. Better performance of the trained neural network 600 may be achieved by additionally incorporating this semantic information into the input. For example, pre-molar teeth have a high tendency to comprise plaque. The behavior of the trained neural network 600 may be adapted by having this information incorporated into the input. For example, numerical representation of the tooth 102 serving as the input 601 to the trained neural network 600 may be modified such that it reflects the fact that the tooth 102 is a pre-molar tooth. The exact way of how this numerical modification takes place may be learned by the trained neural network 600.

It has been found out that advantageously, by inputting natural color information and/or geometric information, associated with the at least one tooth surface of the dental situation, into the trained neural network 600, it may be possible to detect plaque presence value and/or plaque thickness value. Additionally, dental calculus, stains or food debris can be detected due to a distinctive coloration exhibited.

Thereby, the method according to an embodiment may comprise generating the input 601 for the trained neural network 600 from the received digital 3D model 101. Depending on the type of the trained neural network 600, the input 601 may be in 3D format or in 2D format. For example, one type of the trained neural network 600 may be capable of processing 2D images generated from the digital 3D model 101 while another type of the trained neural network 600 may be capable of processing the digital 3D model 101 in form of the point cloud or the graph representation. Thus, the received digital 3D model 101 may be adapted for processing by the trained neural network 600.

Once generated, the input 601 may be provided to the trained neural network 600 in order to obtain the output 602 from the trained neural network 600. All information forming the input 601 may be concatenated into a final input tensor.

Further, the method may comprise obtaining an output 605 from the trained neural network 600 based on the provided input 601, wherein the output comprises the plaque presence value and/or the plaque thickness value associated with the at least one tooth surface.

Obtaining the output 605 from the trained neural network 600 based on the provided input 601 may comprise detecting the plaque presence value and/or the plaque thickness value in the provided input 601.

The output 605 may be in form of a probability value representing the likelihood that the at least part of the digital 3D model 101 comprises plaque and/or that the detected plaque falls into one of the predefined plaque thickness categories. This probability value may relate to the at least one unit element of the at least part of the digital 3D model 101 such as at least one facet, at least one point, at least one node, at least one pixel or at least one voxel, comprised in the at least part of the digital 3D model 101, depending on the format of the provided input 601.

By obtaining the output 605 in this manner, information about plaque presence and/ or plaque thickness in the dental situation may be obtained from a single digital 3D model 101 of the dental situation.

Obtaining the output 605 from the trained neural network 600 based on the provided input 601 may comprise detecting the plaque presence value and/or the plaque thickness value in the input 601 which may be associated with the at least one tooth surface of the dental situation.

The method according to the disclosure may comprise assigning the plaque presence value and/or the plaque thickness value to the at least part of the digital 3D model 101. In this way, the detected plaque presence value and/or the plaque thickness value may be associated with the corresponding region in the digital 3D model 101 and thereby with the corresponding region of the actual dental situation represented by the digital 3D model 101. Correspondence of the at least part of the digital 3D model 101 and the detected plaque presence value and/or the plaque thickness value may be established by means of an identifier, which may be in form of a number. For example, the plaque presence value and/or the plaque thickness value may be detected for a facet, a point, a voxel, a node and/or a pixel of the input 601. The exact position of such a facet, a point, a voxel, a node and/or a pixel, in the at least part of the digital 3D model 101 may be defined through the identifier. The described assigning step may allow for a correct marking and display of the plaque presence value and/or the plaque thickness value on the digital 3D model 101.

The trained neural network 600 may, in a manner described above, perform a classification task per unit element of the input 601 to classify the input 601 according to plaque presence, for example into tooth surfaces comprising plaque and tooth surfaces without plaque. Additionally or alternatively, plaque thickness may be estimated.

The output 605 from the trained neural network 600 may be in a vector format of fixed size which can be derived from a compact representation of the input 601. Values in the output may comprise at least one probability value representing the likelihood that the input 601 comprises plaque. Additionally or alternatively, values in the output may comprise at least one probability value representing the likelihood that the input 601 falls into one of the predefined plaque thickness categories such as thin plaque, medium plaque, thick plaque.

According to an embodiment, the trained neural network 600 may be a trained convolutional neural network (CNN). The trained CNN may comprise a plurality of convolutional layers capable of capturing low-level features of the input, i.e. obtaining convolved features. Moreover, one or more of pooling layers may be present, responsible for reducing the spatial size of convolved features. Convolutional neural networks may be particularly suitable for processing matrix information such as 2D images.

Another example of the trained neural network 600, particularly suitable for performing classification tasks, is a trained Residual Network (ResNet) or a variation of it.

Example of the trained neural network 600 suitable for performing semantic segmentation tasks is a trained U-Net or a variant of it, for example U-Net++.

In an alternative embodiment, the trained neural network 600 may use a self-attention mechanism to process data as in a transformer network. It may also be possible to use, in combination, convolutions, traditionally from convolution networks, and self-attention, traditionally from transformer networks.

In case of three-dimensional (3D) format of the input 601, such as a point cloud, a suitable neural network architecture may be a PointNet network or a variation of it. The PointNet network is capable of processing input in the 3D format, such as a point cloud.

In an embodiment, obtaining the output 605 from the trained neural network 600 may comprise obtaining a probability matrix, wherein the probability matrix allows assignment of the plaque presence value and/or the plaque thickness value for the at least part of the digital 3D model 101. The probability matrix may comprise probability values associated with each unit element of the input. The probability values may specify probabilities of the plaque presence value and/or the plaque thickness value present in the corresponding input unit element.

The plaque presence value may be a simple binary indication of plaque presence on the at least one facet of the tooth surface. Thus, the trained neural network 600 may be capable of classifying, based on previously learned data, the at least one facet of the tooth surface as comprising plaque or not comprising plaque. Instead of the at least one facet other unit elements of the input may be used such as points, nodes, voxels, pixels.

For example, a flag "PLAQUE" to mark plaque presence may be used or a flag "NO PLAQUE" to mark plaque absence may be used.

Advantageously, information on plaque presence in the dental situation may be obtained from a single digital 3D model 101 of the dental situation.

Alternatively or additionally, by using the trained neural network 600, the plaque thickness value may be estimated. The detected plaque may be classified into one of predefined plaque thickness classes such as "no plaque", "thin plaque", "medium plaque", "thick plaque". "No plaque" plaque class may encompass the plaque thickness values less than 20 micrometers. "Thin plaque" may refer to plaque thickness values between 20 and 100 micrometers. "Medium plaque" may refer to plaque thickness values between 100 and 200 micrometers. "Thick plaque" may refer to plaque thickness values above 200 micrometers. Information on plaque thickness is of clinical importance because different thickness values of plaque require different treatment options. For example, it may be possible to remove a thin layer of plaque, lower than 100 micrometers, by constant tooth brushing. However, thick layers of plaque may require intervention of the dental practitioner. Having information on plaque thickness may allow the dental practitioner to correctly define a treatment for the patient.

A treatment recommendation may be automatically presented to the user, depending on plaque thickness value, as a part of the method according to the disclosure.

Alternatively or additionally, by using the trained neural network 600, presence of pathogenic plaque may be estimated.

The output 605 of the trained neural network 600 may be, in an embodiment, a multi-class parameter comprising the plaque presence value, the plaque thickness value and/or the plaque type.

A method according to an embodiment of the disclosure may comprise detecting the pathogenic plaque on the at least part of the digital 3D model 101 using the fluorescence information and detecting the plaque presence value and/or the plaque thickness value using the trained neural network 600.

It may then be possible to display the digital 3D model 101 with the at least part of the digital 3D model 101 determined to comprise plaque and/or determined to comprise a specific plaque thickness, as realized by using the trained neural network 600, and determined to comprise pathogenic plaque, as determined using the fluorescence information.

In the above-described method, the trained neural network 600 may be used to detect the plaque presence value and/or the plaque thickness value while the fluorescence information may be used, in addition, to assess whether the determined plaque is pathogenic plaque.

According to an example, the method may comprise filtering the output of the trained neural network 600 by using the fluorescence information to identify pathogenic plaque presence on the at least one tooth surface.

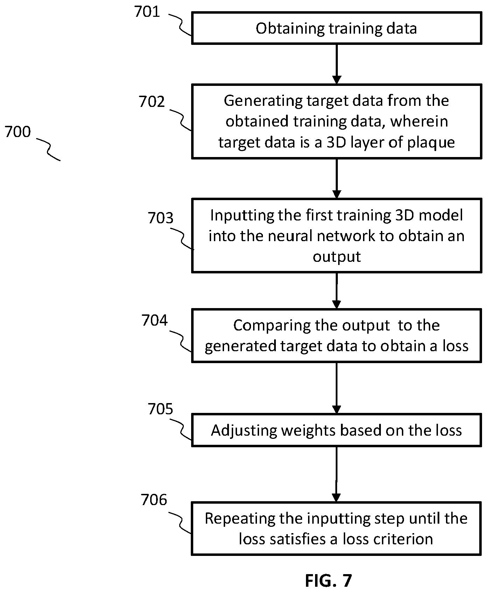

In order to develop the capability of correctly recognizing plaque and/or plaque thickness values on the digital 3D model 101, a neural network may first be trained for that specific task. Training may comprise setting the appropriate weights within the neural network. Once the neural network satisfies training criteria, it may be referred to as the trained neural network. Training is further described with respect to figure 7.

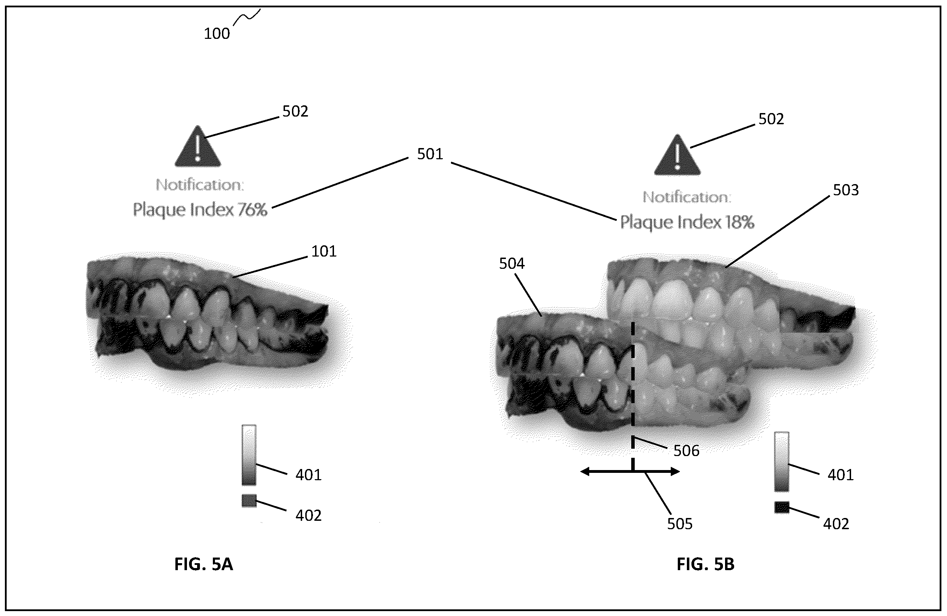

Figure 5A illustrates the digital 3D model 101 where the unit elements have been colored according to detected plaque thickness values. The dependency of the plaque thickness values and colors used to color the corresponding regions of the digital 3D model 101 are shown in the gradient bar 401. Additionally or alternatively, the indicator 402 may indicate the color used to visualize pathogenic plaque on the digital 3D model 101. A value for a plaque index 501 may additionally be displayed.

Additionally or alternatively, a plaque presence indicator 502 may be displayed indicating to the user that plaque has been detected.

This single digital 3D model 101 may also be referred to as a baseline scan.

In order to track the progress of plaque in the dental situation, a further digital 3D model 503 may be received.

The trained neural network 600 according to the disclosure may be utilized on this further 3D model 503 in order to detect plaque thickness values and/or plaque presence. Alternatively, pathogenic plaque presence on the further digital 3D model 503 may be determined from fluorescence information comprised in the further digital 3D model. The further digital 3D model 503 may be obtained from a further scan data obtained from scanning the dental situation at a later time point compared to obtaining the baseline scan. The later time point may be, for example, one year later. The further digital 3D model 503 may also be referred to as a follow-up scan.

The baseline scan 101 and the follow-up scan 503 may then be compared to identify changes in the plaque parameters. The two 3D models 101, 503 may be displayed simultaneously, for example one next to each other. Alternatively, the 3D models 101, 503 may be aligned and superimposed. By aligning the baseline scan 101 and the follow-up scan 503 a superimposed 3D model may be obtained. In order to correctly align the two 3D models 101, 503, it may be necessary to segment each 3D model and subsequently align the corresponding points between the two 3D models 101, 503.

The segmentation process may be performed in different ways, for example based on identification of individual facets or group of facets belonging to a tooth representation. This may allow for identifying objects such as individual teeth and/or surrounding gingiva in the digital 3D models 101, 503 representing the patient's dentition. Therefore, the output of the segmentation process may be individual tooth representations which are usually displayed in form of solid tooth objects, tooth meshes or tooth point clouds.

According to an example, segmenting may comprise use of surface curvatures to identify boundaries of tooth representations. A minimum principal curvature and a mean principal curvature can be used to measure surface property quantitatively. Then, a cutting plane to separate gingiva and teeth part may be produced, based on Principal Component Analysis algorithm (PCA). A curvature threshold value, for example a constant value, can be selected to distinguish tooth boundary regions from the rest of surface.

In another example, segmenting the digital 3D models 101, 503 may comprise use of a harmonic field to identify tooth boundaries. On the digital 3D model, a harmonic field is a scalar attached to each mesh vertex satisfying the condition: $\Delta\Phi=0$, where $\Delta$ is Laplacian operator, subject to Dirichlet boundary constraint conditions. Above equation may be solved, for example using least squares method, to calculate the harmonic field. Segmented tooth representations can then be extracted by selecting optimal isolines connecting datapoints with same value, as tooth representation boundaries.

In yet another example, segmenting the digital 3D model 101 and/or further digital 3D model 503 may comprise use of a segmentation machine learning model. In particular, the digital 3D model may be converted into a series of 2D digital images taken from different perspectives. The segmentation machine learning model may be applied to the series of 2D digital images. For each 2D digital image, classification can be performed to distinguish between different teeth classes and gingiva. After classification of each 2D digital image, backprojection onto the digital 3D model may be performed. This method for segmenting the digital 3D model may be advantageous as the segmentation machine learning model utilizes the series of 2D digital images, overall resulting in fast and accurate classification.

A controller 505 may be arranged in order to allow the user to display the superimposed 3D model 504 as only the digital 3D model 101, only the further digital 3D model 503, or a combination of the two digital 3D models. The controller 505 may allow the user to easily toggle between displaying the different 3D models. The superimposed digital 3D model 504 may comprise a boundary, shown as dashed line 506, which may be translated according to user's manipulation of the controller 505. The boundary 506 may demarcate which part of the superimposed digital 3D model 504 may be shown as the digital 3D model 101 and which part of the superimposed digital 3D model 504 may be shown as the further digital 3D model 503.

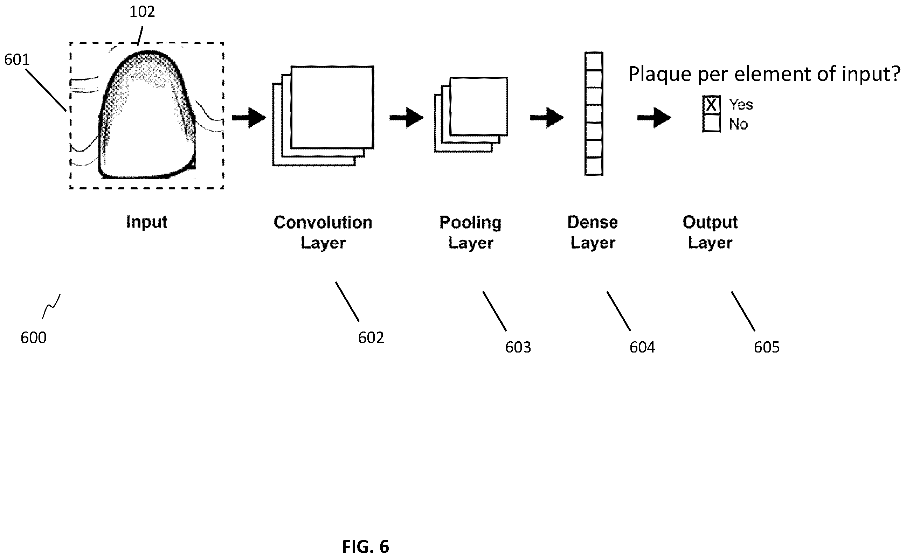

Figure 6 illustrates the trained neural network 600 used in the method according to an embodiment. The trained neural network 600 in this case may be a convolutional neural network and may comprise a plurality of convolution layers 602 capable of capturing low-level features of the input, i.e. obtaining convolved features. Moreover, one or more of pooling layers 603 may be present, responsible for reducing the spatial size of convolved features. Convolutional neural networks may be particularly suitable for processing matrix information such as 2D images.

The input 601 may be in a 2D or 3D format and may comprise the at the at least part of the digital 3D model 101. Figure 6 illustrates the input 601 in the 2D format. The at least part of the digital 3D model 101 may be a 2D image of a tooth 102 which has its labial surface affected by plaque. The at least part of the digital 3D model 101 may alternatively comprise one or more of 2D images of the labial surface of the tooth 102 or at least one pixel of the 2D image of the tooth 102.

The output 605 may comprise the at least one probability value representing the likelihood that the at least part of the digital 3D model 101 comprises plaque and/or falls into one of the predefined plaque thickness classes.

In case of figure 6, the plaque presence value on the at least part of the digital 3D model 101 is determined. The trained neural network 600 in this case may perform a classification task per unit element of the input 601 to classify the input 601 into "Yes" or "No" output class. Alternatively or additionally, the plaque thickness values for plaque present on the tooth 102 may be determined.

Figure 7 illustrates a flowchart of a computer-implemented method 700 for training a neural network for detecting dental plaque.

In an embodiment of the disclosure, a computer-implemented method for training a neural network for detecting dental plaque is disclosed, the method comprising:

- obtaining a training data for the neural network, the training data comprising a first training digital 3D model of a dental situation with plaque present, and a second training digital 3D model of the dental situation with plaque removed,
- generating target data by geometrically subtracting the second training digital 3D model of the dental situation from the first training digital 3D model of the dental situation to obtain a 3D layer of plaque,
- inputting the first training digital 3D model of the dental situation into the neural network to obtain an output from the neural network,
- comparing the output from the neural network to the generated target data to obtain a loss,
- adjusting weights of the neural network based on the obtained loss, and
- repeating the inputting step until the loss satisfies a stopping criterion.

Step 701 of the method 700 illustrates obtaining a training data for the neural network, wherein the training data comprise a first training digital 3D model of a dental situation with plaque present, and a second training digital 3D model of the dental situation with plaque removed.

The training data may comprise a plurality of different digital 3D models referred to as training digital 3D models. This data may be a plurality of intraoral scans obtained by scanning various patients. For example, the training data may comprise the first training digital model of a dental situation comprising plaque. Plaque presence may be determined for example by using the common disclosing agent. Next, the second training digital 3D model of the same dental situation with plaque removed may be obtained. Plaque from the dental situation may be removed, for example by simple tooth brushing, in order to obtain the second training digital 3D model.

Step 702 illustrates generating target data by geometrically subtracting the second training virtual 3D model of the dental situation from the first training virtual 3D model of the dental situation to obtain an isolated 3D layer of plaque. This 3D layer may be used as the target data in order to "teach" the neural network to recognize this plaque layer in the input.

The same process may be repeated for other training digital 3D models associated to other dental situations in order to generate a plurality of different isolated plaque layers. Isolated plaque layers in this case serve as "ground truth".

Step 703 of the method 700 illustrates inputting the first training digital 3D model of the dental situation into the neural network to obtain an output from the neural network.

Step 704 illustrates comparing the output from the neural network to the generated target data to obtain a loss.

Step 705 illustrates adjusting weights of the neural network based on the comparing step. The obtained loss should be minimized, for example by using stochastic gradient descent algorithm (SGD).

Step 706 illustrates repeating the inputting step until the loss satisfies a stopping criterion. The training process is therefore an iterative process. The training process may be stopped when the loss reaches a stopping criterion. For example, the stopping criterion may be a threshold value for the loss. The stopping criterion may also be a number of epochs (training cycles) after which performance metrics cease to increase.

The particular advantage of the mentioned training method is that target data may be generated autonomously, through geometrical subtraction of the training 3D models with and without plaque.

In addition to or alternatively to the training method for determining plaque presence, above-described training method may be used to train a neural network to determine other types of dental plaque parameters such as plaque thickness value or plaque type.

Another example of obtaining ground truth data may be, instead of generating 3D layers of plaque by geometric subtraction, to use manually annotated 3D scans with labels associated with plaque. Even further example of obtaining ground truth data may be to obtain information about plaque presence using the disclosing agent, which may be most accurate from a clinical standpoint. Once the plaque is stained using the disclosing agent, corresponding dental situation may be scanned to obtain a digital form of the dental situation. Obtained digital scan may be segmented and segments with a specific property, for example segments having Red, Green, Blue (RGB) values that correspond to the color of stained plaque, may be selected as the ground truth data. Ground truth data may refer to general plaque presence, but also to plaque thickness and/or type of plaque such as pathogenic plaque.

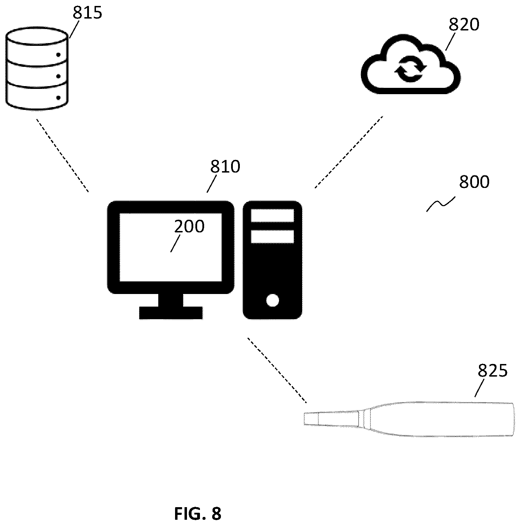

Figure 8 illustrates a dental scanning system 800 which may comprise a computer 810 capable of carrying out any method of the disclosure. The computer may comprise a wired or a wireless interface to a server 815, a cloud server 820 and the intraoral scanner 825. The intraoral scanner 825 may be capable of recording the scan data comprising geometrical information, natural color information and/or fluorescence information associated with the patient's dentition. The intraoral scanner 825 may be equipped with various modules such as a fluorescence module or an infrared module and thereby capable of capturing information relevant for diagnosing dental conditions such as caries, tooth cracks, gingivitis and/or plaque.

The dental scanning system 800 may comprise a data processing device configured to carry out the method according to one or more embodiments of the disclosure. The data processing device may be a part of the computer 810, the server 815 or the cloud server 820.

A non-transitory computer-readable storage medium may be comprised in the dental scanning system 800. The non-transitory computer-readable medium can carry instructions which, when executed by a computer, cause the computer to carry out the method according to one or more embodiments of the disclosure.

A computer program product may be embodied in the non-transitory computer-readable storage medium. The computer program product may comprise instructions which, when executed by a computer, cause the computer to perform the method according to any of the embodiments presented herein.

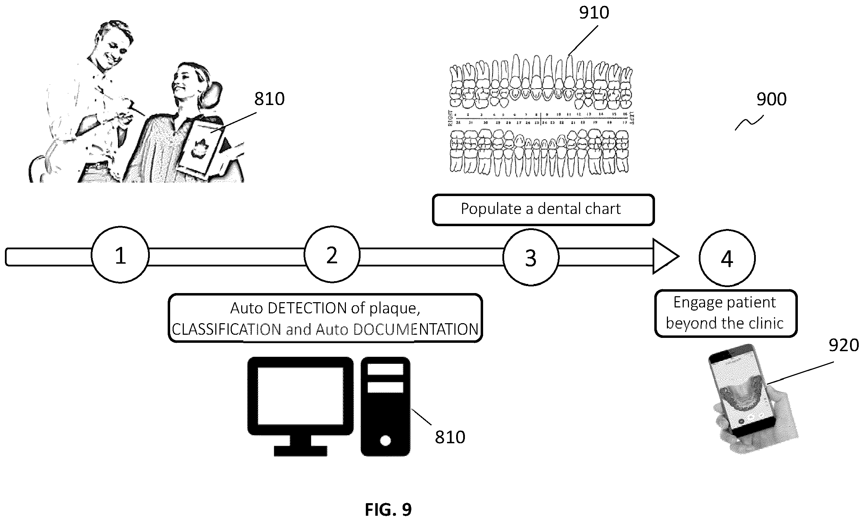

Figure 9 illustrates an exemplary workflow 900 of the patient's visit to the dental practitioner. In step 1 the patient's oral cavity may be scanned using the intraoral scanner 825. In step 1 of the workflow 900 in Figure 9, while scanning, the dental practitioner may be able to see the scanning live on a display unit of the computer 810.

At step 2 of Figure 9, the scan data may be further analyzed utilizing one or more software applications in order to detect dental plaque in the patient's oral cavity. The step 2 of the workflow 900 may utilize software applications (i.e. application modules) that are configured to detect, classify, monitor, predict, prevent, visualize and/or record plaque that may be present in the patient dental situation.

Step 3 of the workflow 900 exemplifies populating a dental chart 910 with information obtained in step 2, such as for example plaque presence, plaque thickness value and/or plaque type in the patient's dental situation. This information may be recorder per tooth surface. The dental chart 910 may be comprised as a part of a wider patient management system.

Furthermore, as illustrated in step 4 of figure 9, it may be possible to connect at least a part of the dental scanning system to a smart phone 920 and thereby enable transferring of relevant information to the patient. The relevant information may comprise information on any dental plaque parameter 301 associated with the patient's dental situation. In this way, engagement with the patient or any other entity using the analyzed scan data beyond the dental clinic may be enabled.

It is to be understood that embodiments may be made, other than those mentioned, and structural and functional modifications may be made without departing from the scope of the present invention.

It should be appreciated that reference throughout this specification to "one embodiment" or "an embodiment" or "an aspect" or features included as "may" means that a particular feature, structure or characteristic described in connection with the embodiments is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

**Claims**

1. A computer-implemented method for detecting dental plaque on a digital 3D model (101) of a dental situation, the method comprising:

   - receiving, by a processor, at least a part of the digital 3D model (101) of the dental situation, wherein the at least part of the digital 3D model (101) comprises fluorescence information associated with fluorescence emitted from dental plaque present on teeth of the dental situation, wherein the fluorescence information comprises a red (R) fluorescence signal value and a green (G) fluorescence signal value of at least one unit element of the at least part of the digital 3D model (101);
   - detecting a dental plaque parameter (301) on the at least part of the digital 3D model (101) based on the fluorescence information, wherein the dental plaque parameter (301) represents pathogenic plaque present on teeth of the dental situation, wherein detecting the dental plaque parameter (301) comprises computing a function of the red (R) fluorescence signal value and the green (G) fluorescence signal value for the at least one unit element of the at least part of the digital 3D model (101);
   - displaying the at least part of the digital 3D model (101) and the detected dental plaque parameter (301).

2. The method according to the previous claim, wherein the function is a difference between the red (R) fluorescence signal value and the green (G) fluorescence signal value for the at least one unit element of the at least part of the digital 3D model (101).

3. The method according to the previous claim 2, further comprising determining that an amplitude of the difference is larger than a noise component of the difference, wherein the noise component comprises a noise signal from both the red (R) fluorescence signal value and the green (G) fluorescence signal value.

4. The method according to the previous claim 1, wherein the function is given as: R-G-(Rh-Gh), wherein Rh is a healthy reference value for red (R) fluorescence and Gh is a healthy reference value for green (G) fluorescence computed per tooth of the digital 3D model (101).

5. The method according to the previous claim 1, wherein the function is a difference of the ratio R/G of the red (R) fluorescence signal value and the green (G) fluorescence signal value, and a ratio Rh/Gh of the healthy reference value for red (R) fluorescence and the healthy reference value for green (G) fluorescence.

6. The method according to claim 4 or 5, wherein the healthy reference value for red (R) fluorescence is obtained by sampling and averaging values of red (R) fluorescence for all vertices belonging to a tooth (102) of the at least part of the digital 3D model (101).

7. The method according to claims 4-6, wherein the healthy reference value for green (G) fluorescence is obtained by sampling and averaging values of green (G) fluorescence for all vertices belonging to the tooth (102) of the at least part of the digital 3D model (101).

8. The method according to the previous claim 7, further wherein the values of green (G) fluorescence for all vertices belonging to the tooth (102) are above a threshold value Gt.

9. The method according to any previous claim, further comprising determining plaque density for at least one tooth surface of the tooth (102) comprising the at least one unit element with the detected dental plaque parameter (301) by counting a number of neighboring unit elements also comprising detected dental plaque parameter (301).

10. The method according to any previous claim, further comprising determining a first number of tooth surfaces comprising the dental plaque parameter (301), determining a second number of tooth surfaces without the dental plaque parameter (301), and determining a ratio of the first number of tooth surfaces to a sum of the first and second number of tooth surfaces to determine a plaque index.

11. The method according to any previous claim, wherein the fluorescence information is comprised in a two-dimensional image wrapped around the at least part of the digital 3D model (101).

12. The method according to any previous claim, further comprising:

   - providing the at least part of the digital 3D model (101) as an input (601) to a trained neural network (600), wherein the at least part of the digital 3D model (101) comprises natural color information and/or geometric information associated with at least one tooth surface of the dental situation;
   - obtaining an output from the trained neural network (600) based on the provided input (601), wherein the output comprises a plaque presence value and/or a plaque thickness value associated with the at least one tooth surface;
   - assigning the plaque presence value and/or the plaque thickness value to the at least part of the digital 3D model (101) .

13. The method according to the previous claim 12, wherein the geometric information associated with the at least one tooth surface comprises a curvature information, a facet normal information, a depth information relating to a virtual camera position and/or an angle between a facet normal and a virtual camera direction.

14. A non-transitory computer-readable medium comprising instructions which, when executed by a computer, cause the computer to carry out the method according to any one or more of the previous claims 1-13.

15. A computer program product comprising instructions which, when the program is executed by a computer, causes the computer to carry out the method of any one or more of the previous claims 1-13.

FIG. 1

EP 4 477 136 A1

201 → Receiving, by a processor, at least a part of a digital 3D model of a dental situation, the at least part of the digital 3D model comprising fluorescence information associated with fluorescence emitted from dental plaque present on teeth of the dental situation

200

202 → Detecting a dental plaque parameter on the at least part of the digital 3D model based on the fluorescence information, wherein the dental plaque parameter represents pathogenic plaque present on teeth of the dental situation

203 → Displaying the at least part of the digital 3D model and the detected dental plaque parameter

**FIG. 2**

FIG. 3A

FIG. 3B

**FIG. 4**

FIG. 5A

FIG. 5B

**FIG. 6**

EP 4 477 136 A1

701 — Obtaining training data

702 — Generating target data from the obtained training data, wherein target data is a 3D layer of plaque

700

703 — Inputting the first training 3D model into the neural network to obtain an output

704 — Comparing the output to the generated target data to obtain a loss

705 — Adjusting weights based on the loss

706 — Repeating the inputting step until the loss satisfies a loss criterion

**FIG. 7**

FIG. 8

910

810

900

Populate a dental chart

1  2  3  4

Auto DETECTION of plaque,
CLASSIFICATION and Auto DOCUMENTATION

Engage patient
beyond the clinic

810

920

**FIG. 9**

## EP 4 477 136 A1

**Europäisches Patentamt**
**European Patent Office**
**Office européen des brevets**

# EUROPEAN SEARCH REPORT

**Application Number**

EP 23 17 9054

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2022/148263 A1 (VANNAHME CHRISTOPH [DK] ET AL) 12 May 2022 (2022-05-12)<br>* abstract *<br>* paragraphs [0001], [0010], [0027], [0031] – [0035], [0072], [0162] – [0167], [0180], [0193], [0240], [0246], [0247]; claims 60,69; figures 13,22 *<br>& WO 2020/182880 A1 (3SHAPE AS [DK]) 17 September 2020 (2020-09-17)<br>----- | 1-15 | INV.<br>A61B5/00<br>G06T7/00 |
| Y | US 2016/125601 A1 (WU YINGQIAN [CN] ET AL) 5 May 2016 (2016-05-05)<br>* abstract *<br>* paragraphs [0069], [0091], [0147]; claims 1,6,16 *<br>* the whole document *<br>----- | 1-15 | |
| A | US 2022/189611 A1 (FARKASH SHAI [IL] ET AL) 16 June 2022 (2022-06-16)<br>* abstract *<br>* paragraphs [0085] – [0091] *<br>----- | 1-15 | |
| A | KR 2019 0019052 A (INSPEKTOR RES SYSTEMS B V [NL]) 26 February 2019 (2019-02-26)<br>* abstract *<br>* paragraphs [0006], [0012], [0019] – [0022]; claims 1,2,12,13 *<br>* the whole document *<br>----- | 1-15 | TECHNICAL FIELDS SEARCHED (IPC)<br><br>G06T<br>G16H<br>A61B |
| A | US 2022/329767 A1 (WANG RUIKANG K [US] ET AL) 13 October 2022 (2022-10-13)<br>* abstract *<br>* paragraphs [0081] – [0084]; claim 1 *<br>----- | 1-15 | |

-/--

The present search report has been drawn up for all claims

| Place of search | Date of completion of the search | Examiner |
|---|---|---|
| The Hague | 28 November 2023 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 23 17 9054

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2021/091671 A1 (COLGATE PALMOLIVE CO [US]; UNIV WASHINGTON [US]) 14 May 2021 (2021-05-14) * abstract * * paragraphs [0026], [0033], [0038], [0039], [0047]; claims 16,27 * | 1-15 | |
| A | US 2018/357766 A1 (VAN DER POEL MIKE [DK] ET AL) 13 December 2018 (2018-12-13) * abstract * * paragraphs [0111], [0112], [0262] - [0267]; claims 1,11 * | 1-15 | |
| A | HIGHAM SUSAN ET AL: "Application of biophysical technologies in dental research", JOURNAL OF APPLIED PHYSICS, AMERICAN INSTITUTE OF PHYSICS, 2 HUNTINGTON QUADRANGLE, MELVILLE, NY 11747, [Online] vol. 105, no. 10, 19 May 2009 (2009-05-19) , pages 102048-1-102048-8, XP012125308, ISSN: 0021-8979, DOI: 10.1063/1.3116633 [retrieved on 2023-11-28] * abstract * * pages 102048-3, left-hand column, paragraph 4 * * pages 102048-1, right-hand column, paragraph 2 * * the whole document * | 1-15 | |

TECHNICAL FIELDS SEARCHED (IPC)

The present search report has been drawn up for all claims

| Place of search | Date of completion of the search | Examiner |
|---|---|---|
| The Hague | 28 November 2023 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 17 9054

This annex lists the patent family members relating to the patent documents cited in the above-mentioned European search report.
The members are as contained in the European Patent Office EDP file on
The European Patent Office is in no way liable for these particulars which are merely given for the purpose of information.

28-11-2023

| Patent document cited in search report | | Publication date | Patent family member(s) | | Publication date |
|---|---|---|---|---|---|
| US 2022148263 | A1 | 12-05-2022 | CN | 113811916 A | 17-12-2021 |
| | | | EP | 3938997 A1 | 19-01-2022 |
| | | | JP | 2022523597 A | 25-04-2022 |
| | | | KR | 20210138652 A | 19-11-2021 |
| | | | US | 2022148263 A1 | 12-05-2022 |
| | | | WO | 2020182880 A1 | 17-09-2020 |
| US 2016125601 | A1 | 05-05-2016 | NONE | | |
| US 2022189611 | A1 | 16-06-2022 | NONE | | |
| KR 20190019052 | A | 26-02-2019 | AU | 2017250458 A1 | 08-11-2018 |
| | | | BR | 112018071196 A2 | 12-02-2019 |
| | | | CA | 3021038 A1 | 19-10-2017 |
| | | | CN | 109475285 A | 15-03-2019 |
| | | | DK | 3442397 T3 | 05-07-2021 |
| | | | EP | 3442397 A1 | 20-02-2019 |
| | | | ES | 2886082 T3 | 16-12-2021 |
| | | | IL | 262401 A | 31-12-2018 |
| | | | KR | 20190019052 A | 26-02-2019 |
| | | | PL | 3442397 T3 | 08-11-2021 |
| | | | RU | 2018139705 A | 13-05-2020 |
| | | | SG | 11201809183X A | 29-11-2018 |
| | | | US | 2019104943 A1 | 11-04-2019 |
| | | | WO | 2017178889 A1 | 19-10-2017 |
| US 2022329767 | A1 | 13-10-2022 | US | 2022329767 A1 | 13-10-2022 |
| | | | WO | 2021067451 A1 | 08-04-2021 |
| WO 2021091671 | A1 | 14-05-2021 | CN | 114630637 A | 14-06-2022 |
| | | | EP | 4054402 A1 | 14-09-2022 |
| | | | JP | 2023501471 A | 18-01-2023 |
| | | | US | 2022409056 A1 | 29-12-2022 |
| | | | WO | 2021091671 A1 | 14-05-2021 |
| US 2018357766 | A1 | 13-12-2018 | BR | 112018011227 A2 | 21-11-2018 |
| | | | CN | 108601636 A | 28-09-2018 |
| | | | EP | 3383311 A1 | 10-10-2018 |
| | | | JP | 2019500095 A | 10-01-2019 |
| | | | JP | 2022122867 A | 23-08-2022 |
| | | | KR | 20180090308 A | 10-08-2018 |
| | | | US | 2018357766 A1 | 13-12-2018 |
| | | | US | 2021106229 A1 | 15-04-2021 |
| | | | US | 2023200655 A1 | 29-06-2023 |
| | | | WO | 2017093563 A1 | 08-06-2017 |

For more details about this annex : see Official Journal of the European Patent Office, No. 12/82